# EUROPÄISCHE PATENTSCHRIFT

(11) **EP 2 382 315 B1**
(45) Veröffentlichungstag und Bekanntmachung des Hinweises auf die Patenterteilung: **23.09.2015**
(21) Anmeldenummer: 09804132.0
(22) Anmeldetag: 23.12.2009
(51) Int. Cl.: C12N 15/10, C12Q 1/68

(54) **NUKLEINSÄUREAUFREINIGUNGSVERFAHREN**
NUCLEIC ACID PURIFICATION METHOD
PROCÉDÉ DE PURIFICATION D'ACIDES NUCLÉIQUES

(30) Priorität: 23.12.2008 DE 102008063001
(43) Veröffentlichungstag der Anmeldung: 02.11.2011
(73) Patentinhaber: Qiagen GmbH, 40724 Hilden (DE)
(72) Erfinder: ERBACHER, Christoph, 42781 Haan (DE); FABIS, Roland, 51375 Leverkusen (DE)
(74) Vertreter: Roth, Carla
(86) Internationale Anmeldenummer: PCT/EP2009/067909
(87) Internationale Veröffentlichungsnummer: WO 2010/072834

(56) Entgegenhaltungen:
- WO-A2-00/69872
- WO-A2-02/48164
- WO-A2-2008/035991
- WO-A2-2008/097342
- US-A1- 2007 197 780

## Beschreibung

Die vorliegende Erfindung betrifft ein Verfahren sowie einen Kit zur Aufreinigung von Nukleinsäuren mit einem nukleinsäurebindenden Trägermaterial. Darüber hinaus werden geeignete Verfahren zur Herstellung entsprechender Trägermaterialien beschrieben.

Verschiedene Verfahren zur Aufreinigung und Isolierung von Nukleinsäuren sind im Stand der Technik bekannt. Diese beinhalten den Einsatz von Phenol-Chloroform, Aussalzverfahren, den Einsatz von Ionenaustauschern sowie Silikapartikel.

Ein bekanntes Verfahren zur Nukleinsäureaufreinigung ist das sog. "Charge-Switch Verfahren". Danach wird eine nukleinsäurebindende Phase, die überwiegend schwach basische Polymere, wie poly Bis-Tris, poly Tris, Polyhistidin, polyhydroxylierte Amine, Chitosan oder Triethanolamin enthalten, bei einem ersten pH-Wert mit einer nukleinsäurehaltigen Probe in Kontakt gebracht, bei der die nukleinsäurebindende Phase eine positive Ladung aufweist. Dies begünstigt die Bindung der negativ geladenen Nukleinsäuren an die Phase. Zur Freisetzung/Elution der Nukleinsäuren wird gemäß dem Charge-Switch Prinzip ein zweiter pH-Wert eingestellt, der höher als der pKs-Wert der nukleinsäurebindende Phase ist, um die positive Ladung zu invertieren, bzw. zu neutralisieren. Die pH-Wert Einstellung über dem pKs-Wert der nukleinsäurebindenden Gruppen der festen Phase fördert die Ablösung der gebundenen Nukleinsäuren von der nukleinsäurebindenden Phase.

Im Stand der Technik sind sowohl lösliche Phasen (siehe bspw. EP 0 707 077) als auch feste Phasen (siehe bspw. WO 99/29703) bekannt. Zur Elution werden verschiedene Lösungen eingesetzt, so bspw. Lösungen, die einen sehr hohen pH-Wert aufweisen oder aber auch biologische Puffer, insbesondere Niedrigsalzpuffer wie bspw. Tris-Puffer.

Die aufgereinigten Nukleinsäuren werden üblicherweise weiterverarbeitet. Diese umfassen bspw. Amplifikationen, wie die Polymerase-Ketten-Reaktion (PCR), enzymatische Reaktionen, wie Restriktion, Ligation, Phosphorylierung, Dephosphorylierung oder RNA-Transkription, Hybrid Capture Assays und Elektrophorese. Diese "downstream" Reaktionen sind oftmals wenig tolerant gegenüber höheren Salzkonzentrationen, so dass vor der Verarbeitung der Nukleinsäuren oftmals ein Entsalzungsschritt erfolgen muss.

Für eine direkte Weiterverwendung der aufgereinigten Nukleinsäuren ohne zusätzliche Umpufferungs- oder Entsalzungsschritte, ist es daher wünschenswert, dass der Elutionspuffer nicht zu salzreich und ferner nicht zu basisch ist.

Bei den Anionenaustauschern steigt jedoch das Elutionsvermögen mit dem pH-Wert und der Salzkonzentration des Elutionspuffers an. Die chromatographischen Anforderungen stehen somit im direkten Widerspruch zu den Anforderungen bezüglich der DNA-Weiterverarbeitung. Der Stand der Technik versucht daher insbesondere die Elution der Nukleinsäuren durch Auswahl geeigneter nukleinsäurebindender Gruppen und pH-Bedingungen zu fördern.

WO 2008/097342 beschreibt ein Verfahren zur Aufreinigung von Nukleinsäuren, bei denen Polykationen wie beispielsweise Spermin nicht-kovalent an einen Träger gebunden werden, um diesen für die Nukleinsäurebindung herzurichten. Als Trägermaterial zur nicht-kovalenten Anwendung der Polykationen werden unter anderem Silikamaterialien genannt. Dieses Dokument hat gemäß einer Ausführungsform zum Ziel, unter milden Bedingungen zu eluieren, damit das Eluat unmittelbar weiterverarbeitet werden kann. Hierfür ist entscheidend, dass die nukleinsäurebindenden Gruppen nicht-kovalent an die Trägermatrix gebunden werden und dass ferner spezifische Waschschritte durchgeführt werden.

WO 00/69872 offenbart ein Verfahren zur Aufreinigung von Nukleinsäuren, bei dem eine speziell funktionalisierte pH abhängige feste Phase zur Bindung der Nukleinsäuren eingesetzt wird. Dieses Dokument beschreibt unter anderem, eine nukleinsäurebindende feste Phase einzusetzen, die mit zwei unterschiedlichen Arten von Liganden funktionalisiert ist, wobei der erste Ligand eine nukleinsäurebindende Gruppe und die zweite Art Ligand eine azidische Gruppe aufweist.

WO 02/48164 offenbart ebenfalls ein Nukleinsäureaufreinigungsverfahren, das auf dem Charge-Switch Prinzip beruht. Die Probe wird bei einem ersten pH-Wert in Kontakt mit einem Material gebracht, das eine ionisierbare Gruppe aufweist, wobei das Material bei dem ersten pH-Wert eine positive Ladung aufweist, so dass die Nukleinsäuren an das Material gebunden werden können. Die Nukleinsäuren werden dann bei einem zweiten, höheren pH-Wert, bei dem die Ladung des Materials negativ, neutral oder weniger positiv ist.

Trotz der bekannten Verfahren zur Aufreinigung von Nukleinsäuren besteht das Bedürfnis, die bestehenden Verfahren zu verbessern, insbesondere die Aufreinigung der Nukleinsäuren so durchzuführen, dass auch eine direkte Weiterverarbeitung der Probe möglich ist.

Der vorliegenden Erfindung liegt daher die Aufgabe zugrunde, ein Verfahren zur Aufreinigung von Nukleinsäuren bereitzustellen, das die direkte Weiterverarbeitung der Probe ermöglicht. Insbesondere soll ein Verfahren zur Verfügung gestellt werden, das die Elution bei niedrigen Salzkonzentrationen erlaubt.

Die vorliegende Erfindung löst diese Aufgabe durch ein Verfahren zu Aufreinigung von Nukleinsäuren, bei dem eine spezielle nukleinsäurebindende Phase eingesetzt wird.

Die nukleinsäurebindende Phase weist nukleinsäurebindende Gruppen A, die einen pKs-Wert von 8 bis 13 aufweisen, sowie ferner bindungsabschwächende ladungsneutrale Gruppen N auf, die bei dem eingesetzten Bindungs-pH Wert und beim eingesetzten Elutions-pH Wert neutral geladen sind, wobei die nukleinsäurebindende Phase ein Trägermaterial aufweist, das mit unterschiedlichen Liganden (Liganden I und Liganden II) versehen ist, wobei die Liganden I wenigstens eine Gruppe A und die Liganden II wenigstens eine Gruppe N aufweisen.

Das Verfahren zur Aufreinigung mit dieser nukleinsäurebindenden Phase weist die folgenden Schritte auf:
(a) Bindung der Nukleinsäuren an die nukleinsäurebindende Phase bei einem pH-Wert, der unterhalb des pK-Wertes der nukleinsäurebindenden Gruppen A liegt (Bindungs-pH Wert);
(b) Elution der Nukleinsäuren bei einem pH-Wert, der oberhalb des Bindungs-pH Wertes liegt (Elutions-pH Wert).

Die vorliegende Erfindung betrifft die Aufreinigung von Nukleinsäuren mittels einer nukleinsäurebindenden Phase, die neben nukleinsäurebindenden Gruppen A auch bindungsabschwächende Gruppen N aufweist. Die nukleinsäurebindenden Gruppen A weisen erfindungsgemäß einen pKs-Wert von 8 bis 13 auf. Die Bindung der Nukleinsäuren erfolgt bei einem pH-Wert unterhalb des pKs-Wertes wenigstens einer dieser Gruppen A. Die Gruppen A nehmen daher ein Proton auf und werden dadurch positiv geladen, was dazu führt, dass die nukleinsäurebindende Phase die negativ geladenen Nukleinsäuren binden kann. Die Elution erfolgt bei einem pH-Wert oberhalb des Bindungs pH-Wertes, wodurch die positive Ladung der nukleinsäurebindenden Phase geringer wird.

Die zusätzlich vorhandenen Gruppen N sind bei dem Bindungs-pH und auch bei dem Elutions-pH Wert ladungsneutral. Die Gruppen N beeinflussen daher in mehrfacher Hinsicht die Bindungsstärke der Gruppen A: (1.) Sie unterbrechen die Anordnung der Gruppen A und beeinflussen damit durch die verringerte Dichte an A-Gruppen die Bindungsstärke den Nukleinsäuren an die nukleinsäurebindende Phase. (2.) Sie können als funktionelle Gruppen, die schwache (z.B. durch van der Waals-Wechselwirkungen) bis mittelstarke Interaktionen (z.B. durch Wasserstoffbrückenbindungen) eingehen können, die Bindungsstärke gezielt modulieren. (3.) Die Gruppen N können mit steigender Größe bzw. Anzahl eine sterische Abschirmung der nukleinsäurebindenden Gruppen A und damit eine Reduktion der Bindungsstärke bewirken. Die Gruppen N bewirken daher eine gleichmäßige, pH-Wert unabhängige Reduzierung der Nukleinsäure-Bindungsstärke. Die dadurch zu einem verringerten Maß gebundenen Nukleinsäuren können leichter von der nukleinsäurebindenden Phase gelöst werden. Die Gruppen N haben die Funktion, die Bindungsstärke bzw. Anzahl der Gruppen A gezielt und kontrolliert zu reduzieren und damit eine gewünschte Häufigkeit in der erfindungsgemäßen nukleinsäurebindenden Phase einzustellen. Je höher der Anteil der Gruppen N ist, desto geringer ist der Anteil an Gruppen A und desto geringer ist die Ladungsdichte und umso schwächer werden Nukleinsäuren an die nukleinsäurebindende Phase gebunden. Das Verhältnis von Gruppen A zu Gruppen N kann durch den Herstellungsprozess gezielt eingestellt werden, so dass man in der Lage ist, die gewünschte Nukleinsäurebindestärke vorab bei dem bzw. durch das Verfahren zur Herstellung der entsprechenden nukleinsäurebindenden Phase (durch z.B. Polymerisation, (Poly-) Kondensation und insbesondere durch die Beschichtung eines Trägermaterials) festzulegen.

Dies hat den erheblichen Vorteil, dass die Elution unter Bedingungen erfolgen kann, die die direkte Weiterverarbeitung der eluierten Nukleinsäuren im Anschluss an die Aufreinigung erlauben. Eine Vielzahl biotechnologischer Methoden, wie bspw. die Amplifikation von Nukleinsäuren (insbesondere mittels PCR), Sequenzierung, Reverse Transkription, Restriktionsanalysen u.a. (siehe oben), sind empfindlich gegenüber Verunreinigungen im Eluat, insbesondere gegenüber hohen Salzkonzentrationen. Das erfindungsgemäße Aufreinigungsverfahren erlaubt in vorteilhafter Weise die Elution bei geringen Salzkonzentrationen, bzw. geringer Ionenstärke, so dass die direkte Weiterverarbeitung in einer sich anschließenden biotechnologischen Methode, insbesondere einer PCR Reaktion ermöglicht wird.

Das Konzept der Abschwächung der Anbindung der Nukleinsäuren durch den Einsatz der Gruppen N führt zu überraschend guten Ergebnissen. Der Stand der Technik setzt für die Verfahrensoptimierung die optimale Bindung der Nukleinsäure voraus und widmet sich dann der Optimierung der Bindungs- und insbesondere Elutionsbedingungen. Eine Abschwächung der Bindungsstärke wurde hingegen in Bezug auf die zu erwartende Ausbeute als nachteilig angesehen, bspw. weil die Nukleinsäuren im Bindungsschritt nur unzureichend an die nukleinsäurebindende Phase binden oder beim Waschen eluiert werden könnten. Unter diesen Gesichtspunkten ist das erfindungsgemäße Konzept der erfolgreichen und schonenden Aufreinigung von Nukleinsäuren trotz Reduzierung der Bindungsstärke durch die Anwesenheit der Gruppen N als äußerst überraschend anzusehen.

Der Anteil der A Gruppen bezogen auf die N-Gruppen kann 1% bis 99%, 1 bis 50%, vorzugsweise 1 % bis 25% betragen.

Die nukleinsäurebindende Phase ist modifiziert, um die Gruppen A und N einzuführen. Erfindungsgemäß gibt es hierzu verschiedene Ausführungsformen, die nachfolgend näher erläutert werden.

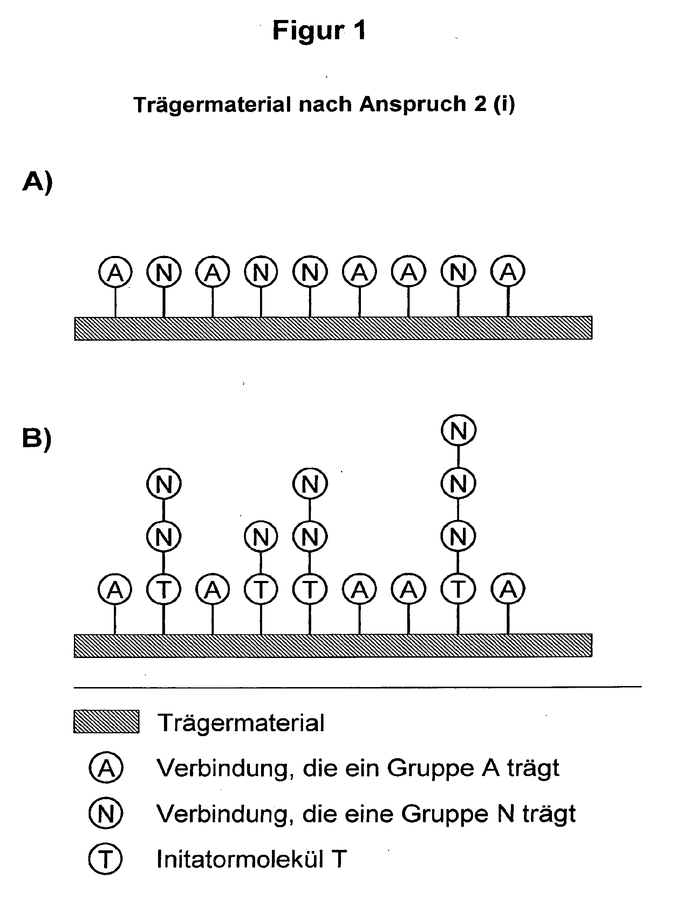

Die nukleinsäurebindende Phase ist mit unterschiedlichen Liganden (Liganden I und Liganden II) versehen, wobei die Liganden I wenigstens eine Gruppe A und die Liganden II wenigstens eine Gruppe N aufweisen. Diese Ausführungsform ist in Figur 1A illustriert. Die Gruppen A und N liegen dann in direkter Nachbarschaft vor, wobei auch Polymercoatings ausgebildet werden können. Durch die Anwesenheit der N-Gruppen tragenden Liganden II nimmt die Anzahl der Gruppen A ab, wodurch die Festigkeit der Bindung der Nukleinsäuren reduziert wird. Dies insbesondere bei Einsatz eines Trägermaterials. Aufgrund der Gruppen N wird daher die Dichte der Gruppen A auf der Trägeroberfläche verringert, was eine Reduktion der Bindungsaffinität zu den Nukleinsäuren zur Folge hat. Als Resultat können die Nukleinsäuren leichter eluiert werden. Der Begriff "Ligand" bzw. "Liganden" bezeichnet insbesondere eine Oberflächenfunktionalisierung, durch die vorzugsweise ein Trägermaterial mit wenigstens einer Gruppe A und/oder einer Gruppe N modifiziert wird, um die erfindungsgemäße nukleinsäurebindende Phase bereitzustellen. Die Liganden I und II liegen vorzugsweise an das Trägermaterial gebunden vor und können bspw. als Monomere, Dimere, Oligomere oder Polymere von reaktiven Einzelmolekülen vorliegen. In einer Variante werden die Gruppen A bzw. die Gruppen N als Bestandteil von mit dem Trägermaterial reagierenden Verbindungen direkt oder über einen Linker bzw. Spacer an ein Trägermaterial gebunden. Gemäß einer bevorzugten Ausführungsform wird diese Modifikation dadurch erzielt, dass das Trägermaterial mit einer die unterschiedlichen Liganden I und II aufweisenden Mischung in Kontakt gebracht wird. Je höher der Anteil an Ligand II bezogen auf Ligand I ist, desto geringer ist die Bindungsstärke an die Nukleinsäure. Dieses Mengenverhältnis kann in einfacher Weise durch ein entsprechendes Mischungsverhältnis der Ligand I - und Ligand II - Edukte bei dem Beschichtungsverfahren gesteuert werden. Einzelheiten sind unten und im Zusammenhang mit der Synthese bzw. dem Verfahren zur Herstellung eines entsprechenden Trägermaterials erläutert. Die Liganden können auch ein Polymercoating ausbilden. Das Trägermaterial kann ferner mit Gruppen A und Initiatormolekülen T ausgestattet sein, wobei ein oder mehrere Gruppen N an die Initiatormoleküle gebunden sind. Diese Ausführungsform ist in Figur 1B) dargestellt. Die Gruppen N liegen dann bezüglich der Oberfläche des Trägermaterials "oberhalb" der Gruppen A vor. Ferner besteht die Möglichkeit auch die Gruppen A über Initiatormoleküle anzubinden.

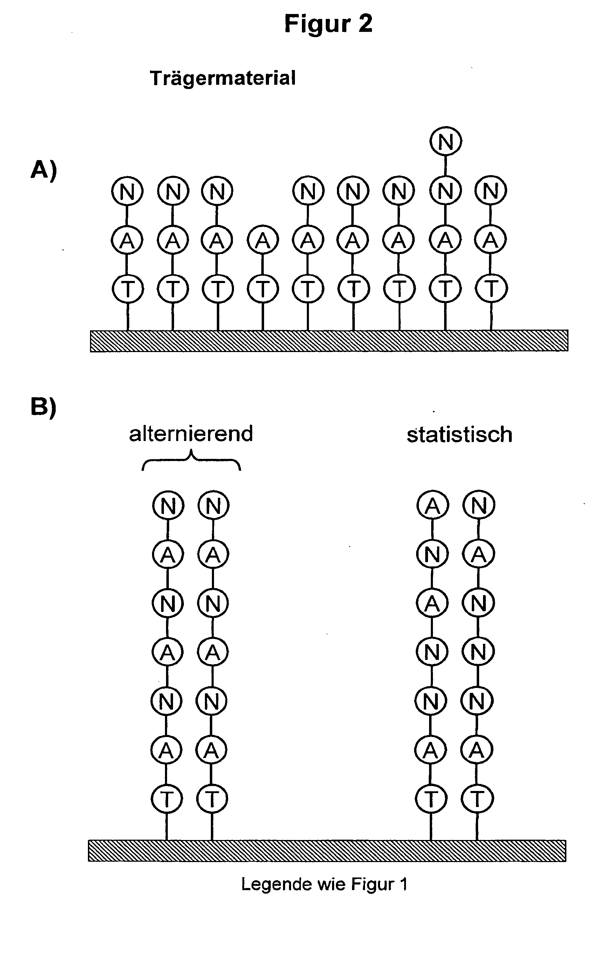

Gemäß eine weiteren Ausführungsform der vorliegenden Erfindung wird das Trägermaterial ferner mit nukleinsäurebindenden Liganden modifiziert, wobei innerhalb eines Liganden eine oder mehrere Gruppen A und eine oder mehrere Gruppen N vorkommen. Diese Ausführungsform ist in Figur 2 näher illustriert. Verschiedene Möglichkeiten existieren, um dieses Konzept zu realisieren.

Gemäß einer Variante ist das Trägermaterial mit Gruppen A (entweder direkt oder bspw. über ein Initiatormolekül T gebunden) ausgestattet, die ein oder mehrere Verbindungen, die eine Gruppe N enthalten, tragen (siehe auch Figur 2A). Bei mehreren Gruppen N können diese auch als unverzweigte Kette oder als verzweigte Baumstruktur oder als entsprechende Mischung vorliegen. Es können hierbei bspw. 1 bis 100 Gruppen N, 1-20, bevorzugt 1-10 und besonders bevorzugt 1-5 Gruppen N pro Ligand vorkommen. Sofern gewünscht können auch noch weitere Gruppen A und N eingeführt werden. Durch diese Verknüpfungsstrategie resultiert eine relativ homogene Bindungsebene mit nahezu reinem A-Gruppen-Besatz und eine oder mehrere Ebenen, die sich nach außen hin anschließen und durch den N-Gruppen-Besatz die Bindungsstärke der Gruppen A verringern.

Gemäß einer weiteren Variante dieser Ausführungsform ist das Trägermaterial mit Gruppen A (entweder direkt oder bspw. über ein Initiatormolekül T gebunden) ausgestattet, die ein Gemisch aus N-Gruppen und A-Gruppen tragen (siehe auch Figur 2B). Hierbei kann zunächst eine oder mehrere Gruppen A an das Trägermaterial gebunden werden. An diese A - haltigen Rumpfliganden werden dann Verbindungen angeknüpft, die wenigstens eine oder mehrere Gruppen N einführen. Durch Steuerung der Reaktionsbedingungen kann jeweils nur eine Gruppe A und N innerhalb des Liganden vorliegen; die Liganden können allerdings auch als Oligomere oder Polymere vorliegen. Bei den als Oligomere und Polymere ausgebildeten Liganden können die Gruppen N statistisch verteilt sein (siehe bspw. Figur 2B, rechte Abbildung), eine alternierende Reihenfolge aufweisen (siehe bspw. Figur 2B, linke Abbildung) oder aber auch als Blockcopolymer angeordnet sein. Auch sind Kombinationen möglich. Geeignete Verfahren zur Ausbildung der entsprechenden A und N- haltigen Funktionalisierungen/Liganden werden im Detail nachfolgend beschrieben.

Gemäß einer weiteren Ausführungsform ist das Trägermaterial mit Gruppen A und N modifiziert, wobei die Gruppen A sterisch durch Verbindungen abgeschirmt werden, die wenigstens eine, vorzugsweise mehrere Gruppen N aufweisen. Auch zu dieser Ausführungsform existieren verschiedene Varianten.

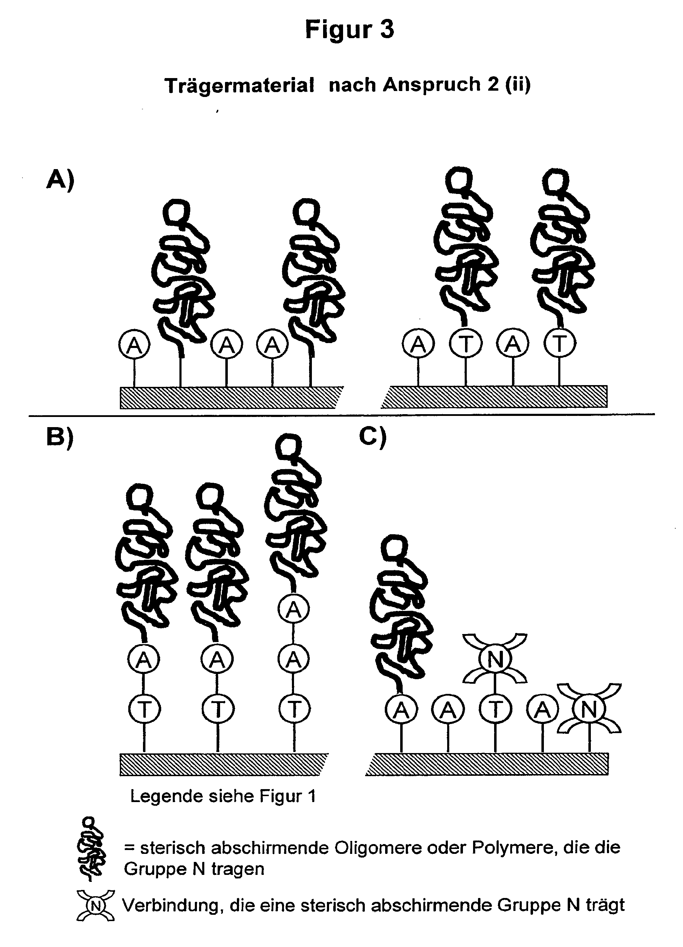

Gemäß einer Variante erfolgt die sterische Abschirmung bevorzugt durch Gruppen N aufweisende Oligomere oder Polymere. Diese können bspw. an die die Gruppe(n) A aufweisenden Liganden gebunden werden (siehe auch Fig. 3B). Sie können jedoch auch benachbart dazu angeordnet sein, sofern die sterische Abschirmung gewährleistet wird (siehe auch Fig. 3A). Die N-Gruppen- aufweisenden Oligomere/Polymere schirmen die Gruppen A durch Bildung von mehr oder weniger geordneten Sekundärstrukturen (z.B. "Random coils" oder Helices) wie eine Art Kappe gegen das Umgebungsmilieu ab und können dadurch die Bindung der Nukleinsäuren abschwächen. Die Oligomere/Polymere können auch aus den Gruppen N gebildet sein und ferner als Block-Copolymere eingeführt werden.

Zur Herstellung dieser Variante kann bspw. in einem ersten Schritt auf dem Trägermaterial Initiatorgruppen T bspw. durch Silanisierung aufgebracht werden. In einem zweiten Schritt können dann bspw. mittels ATRP (Atom transfer radical polymerization) Gruppen A haltige Monomere aufgepfropft werden. Nachdem dieses Monomer abreagiert hat, kann ein zweites Monomer das über Neutralgruppen N verfügt, eingebracht werden. Es entsteht dann ein Copolymer auf dem Träger bestehend aus einem ersten Homopolymer, das Anionenaustauschergruppen trägt, und einem zweiten Homopolymer, das mit dem ersten verknüpft ist und Neutralgruppen N trägt. Über die Menge an dem 2. Monomer, das die Gruppen N trägt, kann die Länge der entstehenden Polymerkette und damit die Stärke der sterischen Abschirmung gesteuert werden.

Das Ausmaß der sterischen Abschirmung kann daher durch die Kettenlänge und die Monomersubstitutionen gesteuert werden. Bevorzugte Kettenlängen für die N-Gruppen aufweisenden Oligomere/Polymere sind n= 10 - 1000, n = 10 bis 500, besonders bevorzugt n = 10 - 100. Zur Ausbildung der Gruppen N tragenden Oligomere/Polymere können die in dieser Anmeldung beschriebenen Monomere eingesetzt werden, die noch im Detail beschrieben werden. Besonders geeignet sind Polyacrylate.

Als bevorzugte Polymere werden Polyacrylate gemäß der folgenden Formel eingesetzt:

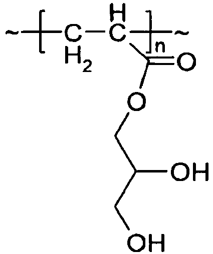

Ein besonders geeignetes Reagenz zur Erzielung der sterischen Abschirmung ist Glycidylmethacrylat, aus dem nach Hydrolyse neutrale Diolgruppen N entstehen. Hierfür kann bspw. durch Silanisierung ein Initiatormolekül T aufgebracht werden. Dann wird ein erstes Monomer, bspw. ein N,N-Dimethylaminopropylmethacrylat bspw. mittels ATRP auf dem Träger polymerisiert. Diese Gruppen werden dann in einem zweiten Polymerisationsschritt sterisch abgeschirmt. Hierzu kann bspw. nachdem die Monomere abreagiert haben ein zweites Monomer eingeführt werden, bspw. Glycidylmethacrylat aus dem nach Hydrolyse neutrale Diolgruppen N entstehen. Der sterische Effekt verhindert den engen Kontakt der Nukleinsäure mit den A Gruppen, so dass diese nicht so stark gebunden und bei geringer Ionenstärke und damit niedrigen Salzkonzentration von der nukleinsäurebindenden Phase eluiert werden können.

Die sterische Abschirmung der Gruppen N kann auch durch "sperrige" Substituenten erzielt werden, zu nennen sind bspw. verzweigte Alkylreste, wie Isopropyl, Diisopropyl, Tertiär-Butyl, aliphatische oder aromatische Ringe, entweder als Kohlenstoffringe oder als Heterozyklen. Diese sterisch hemmenden Gruppen N können auch in Form von Oligomeren/Polymeren eingesetzt werden.

Die sterische Abschirmung kann daher insbesondere gesteuert werden über (i) den Anteil der zusätzlichen eingebrachten Gruppen N; (ii) den Aufbau der Verbindungen, die die Gruppe N tragen; (iii) die Auswahl bzw. Struktur der Gruppe N und (iv) die Kettenlänge der N-Gruppen aufweisenden Oligomere/Polymere.

Eine besonders gute sterische Abschirmung der Gruppe(n) A kann somit durch Gruppen N erfolgen, die im gleichen Liganden vorkommen oder benachbart dazu angeordnet sind. Auch ist eine Bindung an die Gruppe A möglich. Die sterische Abschirmung steigt mit der Größe der die Gruppe(n) N aufweisenden Verbindung und auch mit der Größe der Gruppe N, wobei sowohl eine Erhöhung der Kettenlänge, als auch eine stärkere Verzweigung der Kohlenstoffketten bzw. eine Erhöhung der Ringgröße bei zyklischen Gruppen die sterische Abschirmung erhöht.

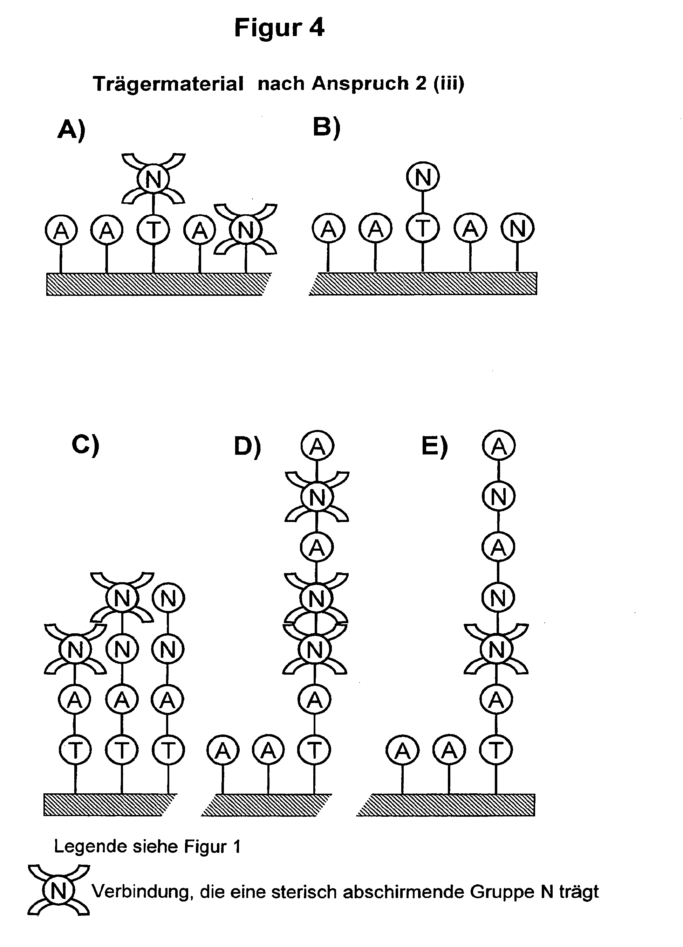

In einer besonderen Ausführungsform ist das Trägermaterial mit einer Kombination sämtlicher oben beschriebener Verknüpfungsstrategien modifiziert. Beispiele sind in Figur 4 gezeigt. So kann das Trägermaterial in direkter Anknüpfung die Gruppen A, die sterisch abschirmenden Gruppen N und Initiatormoleküle T, wobei die Initiatormoleküle noch mit weiteren sterisch abschirmenden Gruppen N ausgestattet sind, aufweisen (siehe bspw. Figur 4 A). Diese Art der Verknüpfung kann bspw. mit den üblichen Gruppen N durchgeführt werden (siehe bspw. Figur 4B). Weiterhin kann das Trägermaterial bspw. mit Gruppen A (direkt oder über ein Initiatormolekül T gebunden) ausgestattet sein und daran angeknüpft ein Gemisch aus N-Gruppen und A-Gruppen tragen, wobei die Gruppen N vollständig oder partiell als sterisch hemmende Gruppe vorliegen (siehe Figur 4D und 4E).

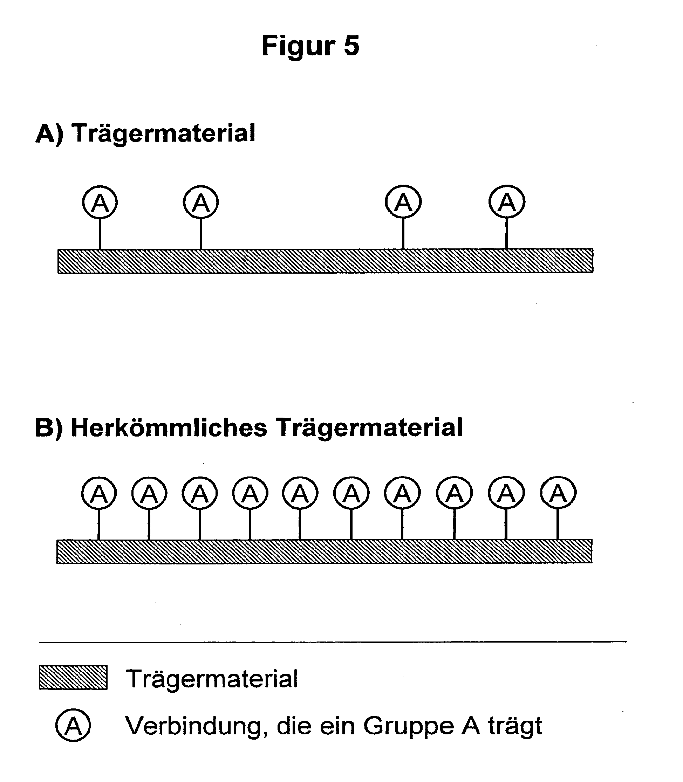

Gemäß einem weiteren Offenbarungsaspekt wird ein Verfahren zur Aufreinigung von Nukleinsäuren mit einer ein Trägermaterial aufweisenden nukleinsäurebindenden Phase durchgeführt, die mit Gruppen A modifiziert ist, wobei nur ein Teil des Trägermaterials mit Gruppen A besetzt ist. Dies bedeutet, dass die übliche Ausstattung mit nukleinsäurebindenden Gruppen deutlich unterschritten wird. Da dadurch weniger nukleinsäurebindende Gruppen A pro Flächeneinheit des Trägermaterials und/oder pro Gramm an Trägermaterial vorkommen, wird die Bindungsstärke der Nukleinsäuren analog der bisherigen Verknüpfungsstrategien reduziert. Figur 5 zeigt eine Ausführungsform dieses Konzepts. Auch diese Ausführungsform ermöglicht daher die Elution der Nukleinsäuren unter Bedingungen, die die direkte Weiterverarbeitung der Nukleinsäuren erlauben, weil insbesondere niedrige Salzkonzentrationen eingesetzt werden können, um die Nukleinsäure von der nukleinsäurebindenden Phase zu eluieren.

Der Unterschuss an A-Gruppen auf dem Trägermaterial kann dadurch erreicht werden, dass die Verbindung, die die Gruppe A einführt, im Verhältnis zum Trägermaterial im Unterschuss eingesetzt wird. Darüber hinaus ist denkbar, dass das Trägermaterial eine geringere Menge an potentiellen Bindungsstellen aufweist, an die die Gruppen A angebunden werden können. Eine weitere Ausführungsform beinhaltet die chemische oder physikalische Vorbehandlung des Trägermaterials, die eine Verringerung der Bindungsstellen oder eine reduzierte Reaktivität der Bindungsstellen bewirkt. Als alternative Strategie kann die Gruppe A - tragende Verbindung im Gemisch mit einer anderen Verbindung, die ebenfalls an das Trägermaterial bindet, aufgebracht werden. Durch die konkurrierende Anbindung sinkt der Anteil der A-Gruppen auf der Oberfläche des Trägermaterials.

Erfindungsgemäß kann die auf einem Unterschuss an A-Gruppen beruhende Ausführungsform mit einer oder mehreren der oben beschriebenen Ausführungsformen zur Modifikation des Trägermaterials mit Gruppen A und N kombiniert werden, wobei gemäß Anspruch 1 die nukleinsäurebindende Phase ein Trägermaterial aufweist, das mit unterschiedlichen Liganden (Liganden I und Liganden II) versehen ist, wobei die Liganden I wenigstens eine Gruppe A und die Liganden II wenigstens eine Gruppe N aufweisen.

Die Ausstattung des Trägers mit Gruppen A im Unterschuss ist insbesondere bei Einsatz eines Silikaträgermaterials von Vorteil, da dieses Silanolgruppen aufweist. Vorzugsweise werden die Gruppen A mittels Silanen oder einem Silangemisch eingeführt. Für die vollständige Umhüllung des Trägers wird eine Mindestmenge an Silan benötigt. Diese Mindestmenge ist definiert durch die spezifische Oberfläche des Trägermaterials. Reicht die Menge an Silan(en) nicht aus, um die Oberfläche des Trägers zu belegen, kann die Ladungsdichte reduziert werden. Durch die Menge an aufzubringendem Silan kann daher die Menge an Gruppen A und dadurch die Bindungsstärke der nukleinsäurebindenden Phase eingestellt werden.

Gemäß einer Ausführungsform weist das Trägermaterial eine Silika-Oberfläche auf, die mit einem Gruppen A aufweisenden Silan beschichtet ist, wobei die Silanmenge 0,1 bis 50 µmol (micromol, hier auch als umol bezeichnet) vorzugsweise 0,1 bis 10 umol beträgt. Entscheidend ist, die Silanmenge so weit zu reduzieren, bis die Elution bei geringeren Ionenstärken bzw. niedrigeren (gewünschten) Salzkonzentrationen möglich ist. Dies kann bspw. experimentell getestet und mittels eines Chromatogramms überprüft werden. Die Silanmenge kann daher vorzugsweise je nach eingesetztem Trägermaterial optimiert werden, um die gewünschten Bindungs/Elutionseigenschaften zu erzielen.

Die nukleinsäurebindende Phase ist eine feste Phase. Zur Herstellung können die Gruppen A, die bindungsabschwächenden Gruppen N und die Initiatormoleküle T sofern vorhanden an ein festes Trägermaterial gebunden werden. Einzelheiten sind nachfolgend noch im Detail beschrieben. Der Einsatz einer festen Phase erleichtert die Abtrennung der gebundenen Nukleinsäuren von der Probe. Gemäß einer Ausführungsform erfolgt daher nach der Bindung der Nukleinsäuren eine Abtrennung der festen Phase bzw. des nicht gebundenen Überstandes.

Als Träger für die nukleinsäurebindenen Gruppen kommen bspw. oxidische Materialien infrage. Es eignen sich hierbei insbesondere Oxide wie Al₂O₃, TiO₂, ZrO₂, Ta₂O₅, SiO₂ und Polykieselsäure, wobei SiO₂ oder Polykieselsäuren als Trägermaterial bevorzugt ist. Als Träger kommen auch organische Polymere wie Polystyrol und seine Derivate, Polyacrylate und -methacrylate, und ihre Derivate oder Polyurethane, Nylon, Polyethylen, Polypropylen, Polybutyliden und Copolymere aus diesen Materialien infrage. Darüber hinaus können diese nukleinsäurebindenden Gruppen auch mit Polysacchariden, insbesondere Hydrogelen wie Agarose, Cellulose, Dextran, Sephadex, Sephacryl, Chitosan verknüpft sein. Des Weiteren können die nukleinsäurebindenden Gruppen auch an anorganische Träger wie bspw. Glas oder Metalloberflächen, wie z. B. Gold, angebunden werden. Magnetische Partikel sind besonders vorteilhaft in der Handhabung. Die nukleinsäurebindenden Gruppen A und/oder die Gruppen N können direkt oder aber über andere chemische Moleküle, bspw. Initiatormoleküle oder Linker an diese Träger gebunden sein. Sie können auch Teil eines größeren Moleküls sein. Sofern das Trägermaterial keine geeigneten funktionellen Gruppen als Bindungsstellen aufweist, können diese in an sich bekannter Weise eingeführt werden.

Weitere Ausführungsformen der Trägermaterialien umfassen nichtmagnetische und magnetische Partikel, Säulenmaterialien, Membranen, sowie Oberflächenbeschichtungen. Ferner seien funktionalisierte Träger wie Röhrchen, Membrane, Vliese, Papier, Reaktionsgefäße wie PCR-Gefäße, "Eppendorf-Tubes", Multiplates, Chips und Mikroarrays genannt.

Gemäß einer Ausführungsform liegt die nukleinsäurebindende Phase sowohl bei der Bindung als auch bei Elution positiv geladen vor.

Ebenfalls offenbart wird eine lösliche nukleinsäurebindende Phase, die Nukleinsäuren nach dem hier beschriebenen Prinzip reversibel bindet. Lösliche Polymere können bspw. alternierend oder statistisch Gruppen A und N aufweisen (siehe bspw. Fig. 2B). Auch können die Seitenketten der A Gruppen durch Gruppen N, insbesondere Alkylreste abgeschirmt sein, genannt seien bspw. Diisopropylreste am Stickstoff. Die im Zusammenhang mit dem Trägermaterial erfolgten Ausführungen gelten für die lösliche nukleinsäurebindende Phase entsprechend.

Die nukleinsäurebindenden Gruppen A sind gemäß einer Ausführungsform Ionenaustauscher; vorzugsweise Anionenaustauscher. Bevorzugte Gruppen A, die sich für die Bindung von Nukleinsäuren bewährt haben, sind Aminogruppen, bevorzugt sind primäre, sekundäre und tertiäre Aminogruppen. Diese können substituiert oder unsubstituiert sein. Darüber hinaus können auch ringförmige Amine, aromatische Amine oder aminofunktionalisierte Heterozyklen eingesetzt werden. Die Amine können Substituenten tragen, bspw. Alkyl, Alkenyl, Alkinyl- oder aromatische Substituenten, darüber hinaus können die Kohlenwasserstoffketten auch zu einem Ring geschlossen sein. Die Kohlenwasserstoffketten können auch Heteroatome, wie Sauerstoff, Stickstoff, Schwefel oder Silizium, oder Verzweigungen aufweisen. Wie ausgeführt weisen die Aminogruppen als Gruppen A pKs-Werte von 8 bis 13, vorzugsweise 9 bis 13, besonders bevorzugt von 10 bis 12 auf.

Die Gruppe A kann Bestandteil einer Verbindung sein, die durch Polymerisation oder Kondensation ein Oligomer oder Polymer ausbildet und damit insbesondere für die Ausbildung der Liganden geeignet ist. Beispiele für solche kettenbildenden Verbindungen sind aminogruppenhaltige Acrylate wie
N-(3-Aminomethyl)methacrylamid, N-(3-Aminoethyl)methacrylamid,
N-(3-Aminopropyl)methacrylamid, N-(3-Aminoisopropyl)methacrylamid
N,N-Dimethylacrylamid, N,N-Diethylacrylamid, N,N-Diisopropylacrylamid
N,N-(Dimethylamino)ethylacrylamid, N,N-(Dimethylamino)ethylacrylat,
N,N-(Dimethylamino)ethylmethacrylamid,N,N-(Dimethylamino)ethylmethacrylat,
N,N-(Dimethylamino)propylacrylamide, N,N-(Dimethylamino)propylacrylat,
N,N-(Dimethylamino)propylmethacrylamide,N,N-(Dimethylamino)propylmethacrylat,
N,N-(Diethylamino)ethylacrylamid, N,N-(Diethylamino)ethylacrylat,
N,N-(Diethylamino)ethylmethacrylamid, N,N-(Diethylamino)ethylmethacrylat,
N, N-(Diethylamino)propylacrylamide, N, N-(Diethylamino)propylacrylat,
N,N-(Diethylamino)propylmethacrylamide,N,N-(Diethylamino)propylmethacrylat,
N,N-(Diisopropylamino)ethylacrylamid, N,N-(Diisopropylamino)ethylacrylat,
N,N-(Diisopropylamino)ethylmethacrylamid, N,N-(Diisopropylamino)ethylmethacrylat,
N,N-(Diisopropylamino)propylacrylamide,N,N-(Diisopropylamino)propylacrylat,
N,N-(Dimethylamino)propylmethacrylamide,N,N-(Dimethylamino)propylmethacrylat,
2-(Dimethylamino)ethylmethacylat (DMAEMA) und 2-(Diisopropylamino)ethylmethacrylat.

Hiervon ist das N,N-(Dimethylamino)propylmethacrylat besonders bevorzugt.

Die Gruppe A kann in einem Silan, bevorzugt in einem Reaktivsilan vorliegen. Als Reaktivsilane werden Verbindungen bezeichnet, die hydrolytisch instabile Si-Bindungen aufweisen, bspw. Si-N oder Si-O Bindungen. Beispiele für Reaktivsilane, die wenigstens eine Gruppe A enthalten, sind: 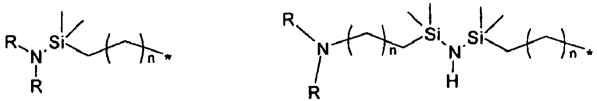 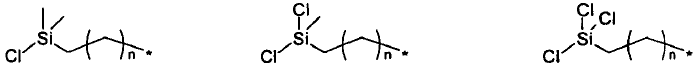 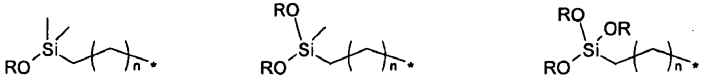 wobei
n: 1 bis 5
R eine C1 bis C6, vorzugsweise eine C1 bis C3 Alkylgruppe ist; und
* Amino-, Aminomethyl-, Aminoethyl-, Aminopropyl-, Dimethylamino-, Diethylamino-, Diisopropylamino-, Dipropylamino-, Diethanolamino-, Dipropanolamino-, Diethylentriamin-, Triethylentetramin-, Tetraethylenpentamin, Etheramin, Polyetheramin, 4-Diisobutylamino-I-butan, 6-Dipropylamino-I-hexan ist.

Entsprechende Reaktivsilane können zur Einführung der Gruppen A eingesetzt werden. Besonders bevorzugt sind Diethylaminopropyltrimethoxysilan (DEAPS), Dimethylamino-propyltrimethoxysilan und N,N-Diisopropylaminopropyltrimethoxysilan.

Gemäß einer Ausführungsform der vorliegenden Erfindung erfolgt die Bindung der Nukleinsäuren bei einem pH-Wert von 2 bis 8, vorzugsweise 4 bis 7,5. Diese Angabe bezieht sich auf den pH-Wert während der Bindung und damit in der Probe. Das erfindungsgemäße Verfahren ist je nach Gestaltung der nukleinsäurebindenden Phase daher auch bei sehr schonenden Bedingungen durchführbar, und kann nahezu im neutralen Bereich durchgeführt werden kann. Aufgrund der Tatsache, dass die protonierbaren Gruppen der nukleinsäurebindenden Phase einen pKs-Wert von 8 bis 13, vorzugsweise 9 bis 13 und besonders bevorzugt 10 bis 12 aufweisen, liegen diese selbst bei relativ neutralen pH-Werten ausreichend positiv geladen vor, um die effektive Anbindung der Nukleinsäuren zu erlauben. Daher kann die Bindung - sofern gewünscht - unter sehr schonenden Bedingungen erfolgen.

Der Bindung kann je nach Ausgangsmaterial wenigstens ein üblicher Lyseschritt vorgeschaltet sein, um die Nukleinsäuren freizusetzen.

Ein weiterer wesentlicher Schritt des vorliegenden Verfahrens ist die Elution der Nukleinsäuren. Wie dargelegt, erfolgt die Nukleinsäurefreisetzung bei einem pH-Wert, der oberhalb des Bindungs pH-Wertes liegt. Dies hat zur Folge, dass die Gruppen A bei der Elution weniger positiv geladen vorliegen, wodurch die Freisetzung der Nukleinsäuren begünstigt wird. Gegenüber herkömmlichen Anionaustauschern bewirkt die Anwesenheit der bindungsabschwächenden Gruppen N eine verringerte Bindung der Nukleinsäuren an das Trägermaterial. Dies hat wie oben dargelegt zur Folge, dass die Elution auch bei niedrigen Salzkonzentrationen durchgeführt werden kann.

Der Elutions-pH Wert liegt vorzugsweise unterhalb des pK-Wertes der Gruppen A. Vorzugsweise erfolgt die Elution je nach eingesetzter nukleinsäurebindender Gruppe A bzw. nukleinsäurebindenden Phase bei einem pH-Wert von 8 bis 11, 8 bis 10, vorzugsweise bei einem pH-Wert von 8,0 bis 9, besonders bevorzugt 8,5 bis 9. Grundsätzlich könnten jedoch auch höhere pH-Werte eingesetzt werden. Bei den bevorzugten niedrigen pH-Werten werden jedoch besonders vorteilhafte Ergebnisse erzielt, da die Nukleinsäuren dennoch bei geringen Salzkonzentrationen freigesetzt werden können und die Bedingungen zudem mild sind.

Um die direkte Weiterverarbeitung der isolierten Nukleinsäuren im Elutionspuffer zu ermöglichen, weist dieser wie ausgeführt vorzugsweise eine niedrige Salzkonzentration auf. Dies wird durch die erfindungsgemäße Ausgestaltung der Nukleinsäurebindenden Phase ermöglicht. Die Salzkonzentration liegt daher gemäß einer Ausführungsform bei 1 mM bis 1000 mM, besonders bevorzugt von 1 mM bis 200 mM, 1 mM bis 250 mM, oder 1 mM bis 100 mM. Geeignete Salze können Chloride der Alkali und Erdalkalimetalle oder Ammonium, andere Salze von Mineralsäuren, Acetate, Borate sowie Verbindungen wie Tris, Bis-Tris sowie organische Puffer, wie bspw. MIS, CHAPS, HEPES und ähnliche darstellen. Entsprechendes gilt für den Bindungspuffer. Geeignete Substanzen zur Elution sind darüber hinaus im Stand der Technik bekannt. Die Salzkonzentration ist im Bindungsschritt und im Elutionsschritt unverändert oder wird bei der Elution leicht angehoben. Vorzugsweise wird die Konzentration jedoch nicht derart erhöht, dass die nachfolgenden Reaktionen behindert werden. Weiterhin kann die Temperatur bei Bindung und Elution gleich sein oder bei der Elution angehoben werden.

Um die Aufreinigung zu begünstigen, wird nach der Bindung und vor der Elution der Nukleinsäuren vorzugsweise wenigstens ein Waschschritt durchgeführt. Zum Waschen sind wässrige Lösungen mit niedrigen Salzkonzentrationen und aber auch Wasser bevorzugt. Wenn Salze in den Waschpuffern enthalten sind, so bevorzugt in einer Konzentration von 1 mM bis 1000 mM, besonders bevorzugt von 1 mM bis 200 mM, 1 mM bis 250 mM, oder 1 mM bis 100 mM. Der Puffer kann organische Verbindungen wie Kohlenhydrate und bevorzugt organische Lösungsmittel wie bspw. Alkohole, Polyole, Polyethylenglykole, Ether, Polyether, Dimethylsulfoxid, Aceton oder Acetonitril. Die Waschpuffer sollten jedoch keine störende Mengen der entsprechenden organischen Bestandteile aufweisen, um die Downstream-Applikationen nicht zu behindern.

Als bindungsabschwächenden Gruppen N können ladungsneutrale Gruppen wie Hydroxylgruppen, Diolgruppen, Triolgruppen, Saccharide, Epoxidgruppen, C1-C6 Alkyl-, Alken-, oder Alkingruppen, Polyolgruppen, Ether, Polyether, Halogenide oder Imide dienen. Durch den Einsatz hydrophiler Gruppen N bleibt der Ionentauscher weiterhin gut mit wässrigen Puffern benetzbar. Die Gruppe N kann Bestandteil einer Verbindung sein, die durch Polymerisation oder Kondensation ein Oligomer oder Polymer bildet. Beispiele für Verbindungen durch die Gruppen N eingeführt werden können, sind Acrylate, wie Butylacrylat, Propylacrylat, Ethylacrylat, Methylacrylat, Glycidylmethacrylat, Hydroxyethylmethacrylat (HEMA), Glycidoxypropylmethacrylat, Glycerolmonomethacrylat (Isomerengemisch), Glycolmonomethacrylat und N-Acryloxysuccinimid.

Die Gruppe N kann auch in einem Silan, bevorzugt in einem Reaktivsilan vorliegen. Beispiele für Reaktivsilane, die die Gruppe N enthalten sind: 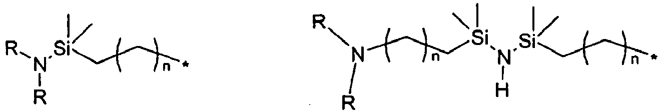 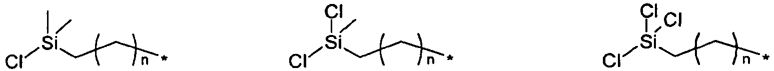 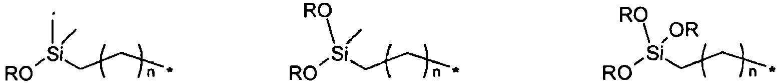 wobei
n= 1-5
R eine C1 bis C6, vorzugsweise C1 bis C3 Alkylgruppe ist;
* Hydroxymethyl-, Hydroxymethyl-, Hydroxypropyl-, Ethandiol-, Propandiol-, Propantriol-, Butantriol, 3-Glycidoxypropyl, Ethylglycidylether, Alkylrest, insbesondere ein C1 bis C4 Alkylrest, Halogenid oder Wasserstoff ist.

Gemäß einer Ausführungsform weist das Trägermaterial Initiatorgruppen T auf, die zumindest teilweise mit Verbindungen funktionalisiert sind, die Gruppen A und/oder N aufweisen. Geeignete Beispiele werden nachfolgend noch im Detail erläutert.

Die Erfindung betrifft ferner die Synthese bzw. ein Verfahren zur Herstellung eines entsprechenden nukleinsäurebindenden Trägermaterials. Hierzu existieren verschiedene Ausführungsformen:
Alternative (A): Bei diesem Verfahren wird das Trägermaterial mit einer Mischung aus wenigstens zwei unterschiedlichen Liganden I und II funktionalisiert, wobei die Liganden I wenigstens eine Gruppe A aufweisen und die Liganden II wenigstens eine Gruppe N aufweisen bzw. darstellen. Einzelheiten zu dieser Ausführungsform wurden bereits oben im Detail erläutert. Wir verweisen auf die obige Offenbarung.
Alternative (B): Bei diesem Verfahren wird in einem ersten Schritt das Trägermaterial mit einer Mischung aus Initiatormolekülen T und Liganden, die wenigstens eine Gruppe A aufweisen, funktionalisiert. Hierbei sind die eine Gruppe A tragenden Verbindungen so ausgewählt oder werden so modifiziert, dass keine weiteren Moleküle angebunden werden können. Im zweiten Schritt werden dann Verbindungen hinzugegeben, die mindestens eine Gruppe N tragen und an die Initiatormoleküle T binden.

Ebenfalls offenbart ist folgende Alternative: Funktionalisierung des Trägermaterials mit nukleinsäurebindenden Liganden, wobei diese innerhalb eines Liganden eine oder mehrere Gruppen A und eine oder mehrere Gruppen N aufweisen. Einzelheiten sind oben erläutert. Vorzugsweise erfolgt in einem ersten Schritt die Anbindung von Initiatormolekülen T an das Trägermaterial. Diese dienen als Ausgangspunkt für das Wachstum einer Copolymerkette. Durch Zugabe von wenigstens eine Gruppe A und/oder oder wenigstens eine Gruppe N aufweisenden Verbindungen und Anbindung dieser an die Initiatormoleküle T. Die Polymerkette wird durch Zugabe weiterer wenigstens eine Gruppe A und/oder wenigstens eine Gruppe N aufweisenden Verbindungen verlängert.

Hierbei können die Polymerisationsschritte auch so gesteuert werden, dass die Gruppen A und N jeweils als Oligomere eingebaut werden, so dass Blockcopolymere entstehen. Ein Bespiel für eine kontrolliert ablaufende Polymerisationsreaktion, die erfindungsgemäß eingesetzt werden kann ist beispielsweise die sogenannte "Atom transfer radical polymerization" (ATRP). Die ATRP zeichnet sich dadurch aus, dass die Konzentration freier Radikale durch Zusatz eines Übergangsmetallkomplexes und in Kombination mit einem Atomtransferprozesses mit einem Organohalogenid soweit erniedrigt wird, dass Kettenabbruchreaktionen, wie Disproportionierung oder Rekombination weitestgehend zurückgedrängt werden.

Alternative (C): Funktionalisierung des Trägermaterials mit Gruppen A und N, wobei die Gruppen A sterisch durch Verbindungen abgeschirmt werden, die wenigstens eine Gruppe N aufweisen. Gemäß einer Ausführungsform dieser Variante erfolgt in einem ersten Schritt die Funktionalisierung des Trägermaterials mit Initiatorgruppen T. Im Anschluss erfolgt die Zugabe von wenigstens eine Gruppe A aufweisenden Verbindungen und Anbindung dieser an die Initiatormoleküle T. In einem weiteren Schritt erfolgt die Abschirmung der Gruppen A durch Anbindung mindestens einer Verbindung, die wenigstens eine Gruppe N aufweist. Vorzugsweise liegen die Gruppen N in Form von Oligomeren/Polymeren vor (siehe oben), die aufgrund ihrer dreidimensionalen Struktur eine sterische Abschirmung der Gruppen A bewirken. Einzelheiten sind oben im Zusammenhang mit dem erfindungsgemäßen Verfahren erläutert.

Ebenfalls offenbart ist folgende Alternative: Hier erfolgt die Funktionalisierung des Trägermaterials mit Gruppen A im Unterschuss. Diese Variante wurde bereits oben im Detail beschrieben. Wir verweisen auf die obige Offenbarung.

Die Alternativen A-C können auch in beliebiger Kombination eingesetzt werden, wobei gemäß Anspruch 1 die nukleinsäurebindende Phase ein Trägermaterial aufweist, das mit unterschiedlichen Liganden (Liganden I und Liganden II) versehen ist, wobei die Liganden I wenigstens eine Gruppe A und die Liganden II wenigstens eine Gruppe N aufweisen. Einzelheiten zu den nukleinsäurebindenden Gruppen A, den Gruppen N sowie den Initiatormolekülen T sind im Detail oben beschrieben und gelten auch im Zusammenhang mit den hier aufgeführten Verfahren zur Trägermodifizierung und kennzeichnen die darin verwendeten Bestandteile. Wir verweisen auf die obige Offenbarung.

Gemäß einer Ausführungsform des Verfahrens zur Funktionalisierung des Trägermaterials mit den Gruppen A und N werden diese Gruppen über monofunktionale, bi- oder trifunktionale Reaktivsilane oder mittels eines Gemisch aus mindestens zwei unterschiedlich funktionalen Reaktivsilanen eingebracht. Als Reaktivsilane können beispielsweise Aminosilane, Disilazane, Chlorsilane oder Alkoxisilane verwendet werden. Der Anteil der A Gruppen bezogen auf die N-Gruppen beträgt 1% bis 99%, 1 bis 50%, vorzugsweise 1 % bis 25%.

Tri- und bifunktionelle Reaktivsilane neigen dazu, durch Polykondensation vernetzte, dicke Schichten auf dem Träger zu bilden. Monofunktionelle Reaktivsilane reagieren dagegen bspw. mit den Silanolgruppen des Trägermaterials unter Bildung von Siloxan (Si-O-Si) Bindungen. Das führt dann zu eher monomolekularen Schichten auf dem Träger. Die Umsetzung des Trägermaterials mit Reaktivsilanen kann in der Gasphase oder in Suspension in einem Lösungsmittel durchgeführt werden, wobei bei Letzterem in Abhängigkeit von der Chemie des Reaktivsilans, organische und bevorzugt wässrige Lösungsmittel verwendet werden können.

Geeignete und bevorzugte Trägermaterialen sind oben im Detail beschrieben. Gemäß einer Ausführungsform des Verfahrens, wird das Trägermaterial zunächst mit Initiatormolekülen T modifiziert und im nächsten Schritt werden die Gruppen A und/oder N in Form von Monomeren eingebracht. Die Initiatormoleküle T können als Anknüpfungsstelle für weitere Verbindungen dienen, die beispielsweise über (Poly-) Kondensation oder Polymerisation, insbesondere eine radikalische Polymerisation wie insbesondere ATRP (Atomtransferradikalpolymerisation) eingeführt werden. Beispiele für Initiatormoleküle T sind: 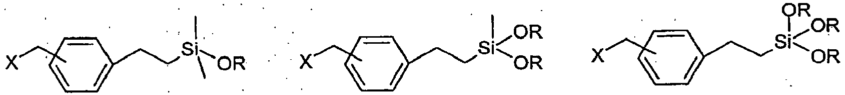 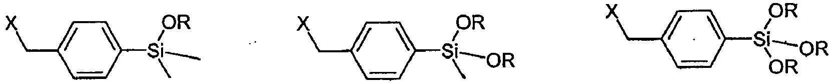 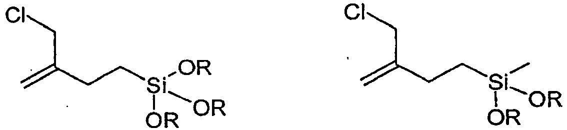 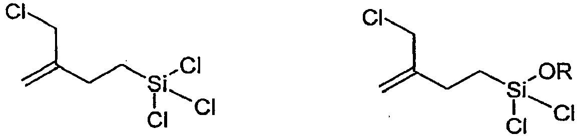 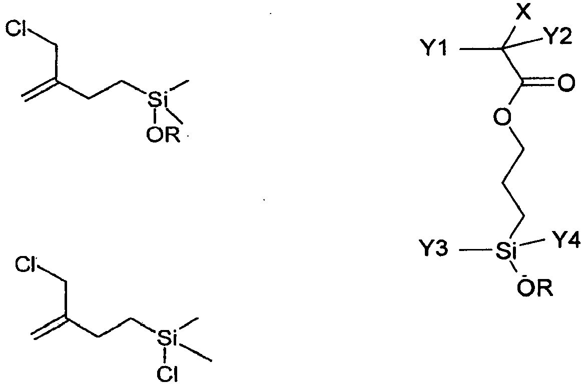
Wobei X = Halogen, insbesondere Cl, Br, I
Y1, Y2, Y3 oder Y4 = unabhängig voneinander R, OR, OH oder H und R = C1 - C3 Alkyl ist.

Vorzugsweise wird 2-(Chloromethyl)allyltrimethoxysilan oder [3-(2-Bromoisobutyryl)propyl]-ethoxydimethylsilan (BPDS) als Initiatormolekül eingesetzt.

Beispiele für Reaktivsilane, durch die nach dem erfindungsgemäßen Verfahren Gruppen A und/oder Gruppen N eingebracht werden können, sind nachfolgend aufgeführt: 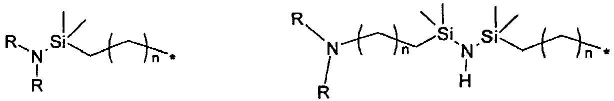 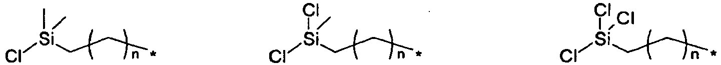 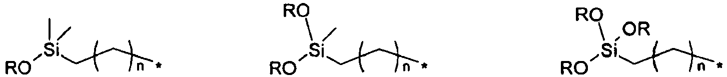 wobei
n= 1-5
R eine C1 bis C6, vorzugsweise C1 bis C3 Alkylgruppe, insbesondere Methyl, Ethyl, Propyl, i-Propyl ist;
* Amino-, Aminomethyl-, Aminoethyl-, Aminopropyl-, Dimethylamino-, Diethylamino-, Diisopropylamino-, Dipropylamino-, Diethanolamino-, Dipropanolamino-, Diethylentriamin-, Triethylentetramin-, Tetraethylenpentamin, Etheramin, Polyetheramin, 4-Diisobutylamino-I-butan, 6-Dipropylamino-I-hexan, Hydroxymethyl-, Hydroxyethyl-, Hydroxypropyl-, Ethandiol-, Propandiol-, Propantriol-, Butantriol, 3-Glycidoxypropyl, Ethylglycidylether, Alkylrest, insbesondere ein C1 bis C4 Alkylrest, insbesondere Methyl, Ethyl, Propyl, i-Propyl, Butyl, i-Butyl, Halogenid oder Wasserstoff ist.

Neben den bereits im Detail beschriebenen Verfahren der gemischten Silanisierung und der Silanisierung im Unterschuss, bestehen weitere Möglichkeiten, die Dichte an Gruppen A auf dem Trägermaterial definiert einzustellen. Wie ausgeführt kann man bspw. ein "grafting - from" Prozess am Trägermaterial durchführen. Dies gelingt beispielsweise durch die Methode der Atomtransferradikalpolymerisation (ATRP), die bereits oben beschrieben wurde. Bei dieser Methode werden zunächst durch Silanisierung Initiatorgruppen T, Gruppen N und/oder Gruppen A auf den Träger aufgebracht. Die Initiatorgruppen T sind bei diesem Prozess entscheidend, da von diesen Gruppen die Initiation des grafting - from Prozesses seinen Ausgang nimmt (Kettenstart).

Beispielsweise gelingt es durch halogenidhaltige Silane, mittels ATRP (Atom Transfer Radical Polymerization), Homopolymere, Copolymere und Block- Copolymere aufwachsen zu lassen. ATRP ist eine besondere Form der Living/Controlled Free Radical Polymerization (LFRP), bei welcher die Konzentration freier Radikale durch Zusatz eines Übergangsmetallkomplexes und in Kombination mit einem Atomtransferprozesses mit einem Organohalogenid soweit erniedrigt wird, dass Kettenabbruchreaktionen, wie Disproportionierung oder Rekombination, weitestgehend zurück gedrängt werden. Dieses Verfahren ermöglicht durch Verwendung von Monomeren und Monomergemischen gezielt die Ladungsdichte der Trägeroberfläche einzustellen und damit die Bindestärke der Nukleinsäuren zu beeinflussen. Durch entsprechende Wahl der Monomere können gezielt die Gruppen A und/oder N aufgebracht werden.

Die entsprechend erzeugten nukleinsäurebindenden Trägermaterialien können insbesondere in dem erfindungsgemäßen Aufreinigungsverfahren zum Einsatz kommen.

Die Erfindung betrifft ferner die Verwendung einer erfindungsgemäßen nukleinsäurebindenden Phase zur Aufreinigung von Nukleinsäuren. Nukleinsäuren im Sinne der Erfindung umfassen insbesondere DNA und RNA, insbesondere genomische DNA, Plasmid-DNA, sowie PCR Fragmente, cDNA, miRNA, siRNA, sowie Oligonukleotide und modifizierte Nukleinsäuren wie bspw. PMA oder LMA. Es können auch virale oder bakterielle RNA sowie DNA oder Nukleinsäuren aus menschlichen, tierischen oder pflanzlichen Quellen aufgereinigt werden. Des Weiteren kommen für eine erfindungsgemäße Aufreinigung auch DNA/RNA Hybride in Frage sowie modifizierte Nukleinsäuren.

Ebenfalls offenbart ist ein Kit zur Aufreinigung von Nukleinsäuren, das dadurch gekennzeichnet ist, dass es ein erfindungsgemäßes nukleinsäurebindendes Trägermaterial aufweist, das nukleinsäurebindende Gruppen A mit wenigstens einer protonierbaren Gruppe aufweist, die einen pKs-Wert von 8 bis 13, vorzugsweise 9 bis 13, besonders bevorzugt 10 bis 12 aufweisen. Einzelheiten zu dem erfindungsgemäßen Trägermaterial und seiner Funktionalisierung sind oben im Detail ausgeführt; wir verweisen auf obige Ausführungen.

Das Kit kann ferner Bindungs-, Wasch- und/oder Elutionspuffer aufweisen, wie sie bspw. oben im Zusammenhang mit dem Aufreinigungsverfahren beschrieben wurden. Darüber hinaus kann es Lyse- und Neutralisierungspuffer aufweisen.

Gemäß einer Ausführungsform weist das Kit einen Bindungspuffer auf, der vorzugsweise wenigstens eines der nachfolgenden Merkmale aufweist:
(a) einen pH-Wert von 1 bis 13; und/oder
(b) eine Salzkonzentration von 1 mM bis 1000 mM, besonders bevorzugt von 1 mM bis 200 mM, 1 mM bis 250 mM, oder 1 mM bis 100 mM.

Die Vorteile der entsprechenden Merkmale wurden oben bereits im Zusammenhang mit dem Verfahren erläutert, wir verweisen auf die obige Offenbarung.

Gemäß einer Ausführungsform weist das offenbarte Kit ferner einen Waschpuffer auf, der vorzugsweise wenigstens eines der nachfolgenden Merkmale aufweist:
(a) einen pH-Wert von 2 bis 7, vorzugsweise 4 bis 7;
   und/oder
(b) eine Salzkonzentration von 1 mM bis 1000 mM, besonders bevorzugt von 1 mM bis 800 mM, 1 mM bis 600 mM; und/oder
(c) er ist ausgewählt aus der Gruppe bestehend aus Wasser, biologische Puffer, organische Puffer, insbesondere Tris, Tris-Bis, MIS, MOPS, CHAPS und HEPES.

Gemäß einer weiteren Ausführungsform weist das Kit ferner einen Elutionspuffer auf, der vorzugsweise wenigstens eines der nachfolgenden Merkmale aufweist:
(a) einen pH-Wert von 8 bis 10, bevorzugt 8 bis 9
   und/oder
(b) eine Salzkonzentration von 1 mM bis 1000 mM, besonders bevorzugt von 1 mM bis 200 mM, 1 mM bis 250 mM, oder 1 mM bis 100 mM; und/oder
(c) er ist ausgewählt aus der Gruppe bestehend aus Wasser, biologische Puffer, organische Puffer, insbesondere Tris, Tris-Bis, MIS, MOPS, CHAPS und HEPES.

Einzelheiten zur nukleinsäurebindenden Phase sowie den Elutionsbedingungen sind im Detail oben beschrieben und gelten auch im Zusammenhang mit dem offenbarten Kit und kennzeichnen die darin verwendeten Bestandteile/Puffer. Wir verweisen auf die obige Offenbarung.

Die offenbarten Kits können insbesondere im Rahmen des erfindungsgemäßen Verfahrens zur Anwendung kommen. Die vorliegenden Verfahren, Kits sowie nukleinsäurebindenden festen Phasen können insbesondere im Bereich der Molekularbiologie, der molekularen Diagnostik, in der Forensik, in der Lebensmittelanalytik sowie im Applied Testing eingesetzt werden. Die Anwendung des offenbarten Kits erlaubt die unmittelbare Weiterverarbeitung der aufgereinigten Nukleinsäuren in "downstream" Applikationen, insbesondere in einer PCR Reaktion.

Durch Wahl/Kombination der beschriebenen Parameter, insbesondere der die Elution fördernden bindungsabschwächenden Gruppen N, und der Reduzierung der nukleinsäurebindenden Gruppen kann der pH-Wert der nukleinsäurebindenden Phase in Bezug auf die Elutionsbedingungen optimal eingestellt werden. Entsprechend kann das Elutionsprofil der nukleinsäurebindenden Phase, insbesondere die Salzkonzentration und der Elutions pH-Wert gesteuert bzw. eingestellt werden.

Nukleinsäuren, die mit den erfindungsgemäßen Systemen aufgereinigt werden können, können in Körperflüssigkeiten wie Blut, Urin, Stuhl, Speichel, in biologischen Quellen wie Gewebe, Zellen, insbesondere Tierzellen, Humanzellen, Pflanzenzellen, Bakterienzellen und dergleichen, Organen wie Leber, Niere oder Lunge vorliegen, dazu kann die Nukleinsäure aus Trägermaterialien wie Swabs, PapSmears, sowie stabilisierende Medien wie PreServCyt oder Surepath, oder auch aus weiteren Flüssigkeiten, wie z.B. Säften, wässrigen Proben oder allgemein Lebensmitteln gewonnen werden. Darüber hinaus können die Nukleinsäuren aus Pflanzenmaterial, bakteriellen Lysaten, in Paraffin eingebettetem Gewebe, wässrige Lösungen oder Gelen gewonnen werden.

Die eluierten Nukleinsäuren können vorzugsweise direkt weiterverarbeitet werden, so bspw. im Rahmen einer PCR, RT-PCR, einem Restriktionsverdau oder einer Transkription zum Einsatz kommen. Eine weitere Aufreinigung ist nicht erforderlich, sofern die Elutionspuffer wie oben beschrieben ausgestaltet sind und vorzugsweise eine niedrige Salzkonzentration aufweisen.

### FIGUREN

Die Figuren zeigen:
**Figur 1****.** Schematische Übersicht über die Ausstattung des Trägermaterials mit den nukleinsäurebindenden Gruppen A und den bindungsschwächenden Gruppen N, wobei diese auf separaten Liganden vorkommen (siehe auch Anspruch 2(i)).
**Figur 2****.** Schematische Übersicht über die Ausstattung des Trägermaterials mit den nukleinsäurebindenden Gruppen A und den bindungeshemmenden Gruppen N, wobei diese gemischt innerhalb eines Liganden vorkommen. (A) stellt ein Trägermaterial dar, das zuerst mit Gruppen A und dann mit Gruppen N ausgestattet worden ist; (B) stellt eine alternierende Abfolge von A und N innerhalb eines Liganden dar.
**Figur 3****.** Schematische Übersicht über die Ausstattung des Trägermaterials mit den nukleinsäurebindenden Gruppen A und den sterisch abschirmenden Gruppen N, wobei diese gemäß den in Figur 1 und 2 dargestellten Schemata mit den nukleinsäurebindenden Gruppen A kombiniert werden können. Siehe Anspruch 2 (ii).
**Figur 4****.** Schematische Übersicht über die beispielhafte Ausstattung des Trägermaterials mit den nukleinsäurebindenden Gruppen A und den bindungshemmenden und/oder sterisch abschirmenden Gruppen N, wobei diese aus einer Kombination der in Figur 1 bis 3 dargestellten Schemata gebildet werden. Siehe Anspruch 2 (iii).
**Figur 5****.** Schematische Übersicht über die Ausstattung des Trägermaterials mit einem Unterschuss an nukleinsäurebindenden Gruppen A.
**Figur 6****.** Elutionsprofil gemäß Beispiel A 3 a) bei verschiedenen pH-Werten.
**Figur 7****.** Elutionsprofil gemäß Beispiel A 3 b) bei verschiedenen pH-Werten.
**Figur 8****.** Elutions-Diagramm für die gemäß Beispiel C.1) hergestellten Kieselgele.

Die vorliegende Erfindung soll nachfolgend anhand von einigen Beispielen erläutert werden. Diese sind nicht beschränkend, stellen jedoch bevorzugte Ausführungsformen der vorliegenden Erfindung dar. Beispiele, die nicht durch die Ansprüche erfasst sind, dienen lediglich zu Illustrationszwecken. Darüber hinaus werden sämtliche der hierin genannten Referenzen zum Gegenstand der Offenbarung gemacht.

### BEISPIELE

Im Rahmen der Experimente wurde Plasmid-DNA als Modellsysteme für Nukleinsäuren verwendet.

### A) Modifizierung eines Trägermaterials mit benachbarten Gruppen A und N

### A.1) Silanisierung von Kieselgel mit 2-(Chloromethyl)allyltrimethoxysilan und DEAPS im molaren Verhältnis von 1:1 (AAK01-10)

### Materialien

Trägermaterial: Silika/Kieselgel mit einer Porengröße von ca. 150nm. Die spezifische Oberfläche beträgt ca. 25m²/g.

Beschichtungsreagenzien: 2-(Chloromethyl)allyltrimethoxysilan, DEAPS; QSP1-Puffer (saure Acetatpuffer).

### Herstellungsvorschrift

In einem Dreihalskolben wurden 70 ml Wasser, 2.5 ml QSP1-Puffer (QIAGEN), 322 µl 2-(Chloromethyl)allyltrimethoxysilan und 413 µl DEAPS mit resultierendem pH 5.3 vorgelegt und 18g Kieselgel zugegeben. Die Mischung wurde unter Rühren innerhalb von 20 min auf 95°C erhitzt, für 4 Stunden bei dieser Temperatur weitergerührt und dann 1 h unter Rühren abgekühlt. Das Kieselgel wurde über eine P3 Fritte abgetrennt und nacheinander mit 32,3 g Tris/NaCl-Puffer, 2-mal mit 30 ml VE-Wasser, 2-mal mit 35 ml Methanol und abschließend mit 30 ml Methanol gewaschen. Das fertige Trägermaterial **AAK01-10** wurde über Nacht bei 125°C getrocknet.

Zum Vergleich wurde das identische Trägermaterial ausschließlich mit der die Gruppe A tragenden Verbindung DEAPS modifiziert. In einem Dreihalskolben wurden 70 ml Wasser, 2.5 ml QSP1-Puffer, und 825 µl DEAPS mit resultierendem pH 5.5 vorgelegt und 18 g Kieselgel zugegeben. Die Mischung wurde unter Rühren innerhalb von 20 min auf 95°C erhitzt, für 4 Stunden bei dieser Temperatur weitergerührt und dann 1 h unter Rühren abgekühlt. Das Kieselgel wurde über eine P3 Fritte abgetrennt und nacheinander mit 32,3 g Tris/NaCl-Puffer, 2-mal mit 30 ml VE-Wasser, 2-mal mit 35 ml Methanol und abschließend mit 30 ml Methanol gewaschen. Das fertige Trägermaterial **AAK01-30** wurde über Nacht bei 125°C getrocknet.

### Aufreinigung von Plasmid DNA mit dem modifizierten Trägermaterial AAK01-10 unter Bestimmung des Elutionspunktes

Die Bindung wurde in einem Puffer 50mM Tris -HCl pH7.0, 15% Ethanol durchgeführt. Zur Elution wurde innerhalb von 23 min ein Stufengradient von 0% zu 100% Puffer B (50mM Tris-HCl, pH 7.0, 15% Ethanol, 2M NaCl) gefahren und die Menge der eluierten DNA kontinuierlich mittels UV-Spektroskopie bestimmt. Die NaCl-Konzentration, bei der erstmalig signifikante Mengen an DNA eluiert wurde, wurde als Elutionspunkt festgehalten. Dabei zeigte sich, dass die Einführung der Gruppen N den Elutionspunkt signifikant senken.

Das ausschließlich mit dem protonierbaren Gruppen A (DEAPS) beschichtete Trägermaterial (AAK01-30) zeigt bei einem pH-Wert von 7.0 den Elutionspunkt bei 1600mM NaCl. Es werden also recht hohe Salzkonzentrationen benötigt, um die Nukleinsäuren zu eluieren. Für das gemischt A/N-modifizierte Kieselgel AAK01-10 ergibt sich der Elutionspunkt bei ∼700 mM NaCl. Die zur Elution notwendige Ionenstärke wurde somit um mehr als 50% reduziert.

### A.2) Silanisierung des A/N-modifizierten Kieselgels mit Hydroxyethylmethacrylat (HEMA) (AAK01-11)

### Materialien

Ausgangsmaterial: modifiziertes Kieselgel AAK01-10 (siehe Vorschrift A.1)
Beschichtungsreagenzien: Hydroxyethylmethacrylat (HEMA).

### Herstellungsvorschrift

Mit Hilfe der durch Cu(I)-katalysierten Atomtransferradikalpolymerisation (ATRP) wird an das bereits gebundene Chlorsilan HEMA Oligomere aufgebaut.

Im Reaktionskolben werden 20ml HEMA, die vorab über Al₂O₃ aktiviert wurden, mit 20 ml VE-Wasser 30 min mit Argon gespült, dann 68 mg CuCl₂, 46 mg CuBr₂ und 313 mg Bispyridin hinzugegeben und nach Untermischen 10 g AAK01-20 hinzugefügt. Die Mischung wird für 4 Stunden bei Raumtemperatur gerührt,und direkt danach über eine P3 Glasfilterfritte abgesaugt. Auf der Fritte wird das Material zunächst mehrfach mit 100 mM NaCl/100 mM EDTA-Puffer gewaschen, dann mit VE-Wasser, einmal mit 8 ml Methanol, und zweimal mit 7 ml Methanol gewaschen. Das fertige Trägermaterial **AAK01-11** wurde für 14 Stunden bei 60°C getrocknet.

Zum Vergleich wurde das mit DEAPS modifizierte Trägermaterial AAK01-30 in gleicher Weise mit HEMA umgesetzt und aufgearbeitet. Das Endprodukt dieser Reaktion wurde als AAK01-31 bezeichnet. Da das DEAPS als funktionelle Gruppe nur ein tertiäres Amin, allerdings kein polymerisationsfähige Allylgruppe trägt, sollte keine HEMA-Ankopplung erfolgen.

### Aufreinigung von Plasmid DNA mit dem modifizierten Trägermaterial AAK01-11 unter Bestimmung des Elutionspunktes

Die Versuche wurden wie unter A.1 beschrieben durchgeführt. Das ausschließlich mit dem protonierbaren Gruppen (DEAPS) beschichtete Trägermaterial AAK01-31 zeigt bei einem pH-Wert von 7.0 den Elutionspunkt bei 1500 mM NaCl. Es werden also recht hohe Salzkonzentrationen benötigt, um die Nukleinsäuren zu eluieren. Für das mit HEMA-modifizierte Kieselgel AK01-11 ergibt sich der Elutionspunkt bei ∼400 mM NaCl. Die zur Elution notwendige Ionenstärke wurde somit um nahezu 75% reduziert.

### A.3) Silanisierung von Silikabeads mit Silanen in verschiedenen molaren Verhältnissen.

### a) FF-Silikabeads

Der nachfolgende Versuchsaufbau wurde gewählt. Als Trägermaterial wurden FF-Silikabeads eingesetzt.

| | **Silan pro g Trägermaterial [µmol]** | **Verhältnis der Silane zueinander** | | | | **pH-Wert der Elutionspuffers** | **mittels Elutionspuffer* eluierte DNA pro 50mg Beads [µg]** | **restliche gebundene DNA pro 50mg Beads eluiert mit QN**-Puffer eluiert [µg]** |
|---|---|---|---|---|---|---|---|---|
| | | **Silan A** | | **Silan N** | | | | |
| | | [mmol%] | [µmol] | [mmol%] | [µmol] | | | |
| HL09MB | 182,8 | 25,00 | 45,700 | 75,00 | 137,100 | pH 7,50 | 4,7 | 35,6 |
| HL09MB | 182,8 | 25,00 | 45,700 | 75,00 | 137,100 | pH 7,75 | 20,3 | 29,0 |
| HL09MB | 182,8 | 25,00 | 45,700 | 75,00 | 137,100 | pH 8,00 | 37,3 | 17,3 |
| HL09MB | 182,8 | 25,00 | 45,700 | 75,00 | 137,100 | pH 8,25 | 48,1 | 11,0 |
| HL09MB | 182,8 | 25,00 | 45,700 | 75,00 | 137,100 | pH 8,50 | 52,5 | 7,2 |

| | | | | | | | | |
|---|---|---|---|---|---|---|---|---|
| *: Zusammensetzung Elutionspuffer: 20mM Kaliumchlorid; 50mM Tris in Wasser auf pH X eingestellt; **: Zusammensetzung QN-Puffer: 1600mM Natriumchlorid; 50mM Tris 15% Ethanol in Wasser auf pH 7 eingestellt | | | | | | | | |

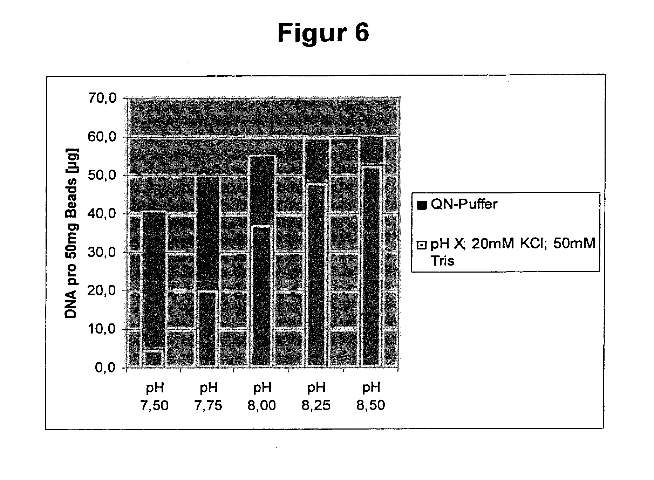

Die Ergebnisse sind in **Fig. 6** gezeigt.

### b) MagAttract Beads G (QIAGEN)

Der nachfolgende Versuchsaufbau wurde gewählt. Als Trägermaterial wurden die magnetischen Silikabeads MagAttract Beads G der Firma QIAGEN eingesetzt:

| | **Silan pro g Trägermaterial [µmol]** | **Verhältnis der Silane zueinander** | | | | **pH-Wert der Elutionspuffers** | **mittels Elutionspuffer* eluierte DNA pro 50mg Beads [µg]** | **restliche gebundene DNA pro 50mg Beads eluiert mit QN**-Puffer eluiert [µg]** |
|---|---|---|---|---|---|---|---|---|
| | | **Silan A** | | **Silan N** | | | | |
| | | [mmol%] | [µmol] | [mmol%] | [µmol] | | | |
| HLG09ST | 182,8 | 25,00 | 45,700 | 75,00 | 137,100 | pH 7,50 | 4,3 | 69,5 |
| HLG09ST | 182,8 | 25,00 | 45,700 | 75,00 | 137,100 | pH 7,75 | 28,6 | 40,9 |
| HLG09ST | 182.8 | 25,00 | 45,700 | 75,00 | 137,100 | pH 8,00 | 50,6 | 15,5 |
| HLG09ST | 182,8 | 25,00 | 45,700 | 75,00 | 137,100 | pH 8,25 | 60,3 | 2,8 |
| HLG09ST | 182,8 | 25,00 | 45,700 | 75,00 | 137,100 | pH 8,50 | 69,0 | 2,7 |

| | | | | | | | | |
|---|---|---|---|---|---|---|---|---|
| *: Zusammensetzung Elutionspuffer 20mM Kaliumchlorid; 50mM Tris in Wasser auf pH X eingestellt; **: Zusammensetzung QN-Puffer: 1600mM Natriumchlorid; 50mM Tris 15% Ethanol in Wasser auf pH 7 eingestellt | | | | | | | | |

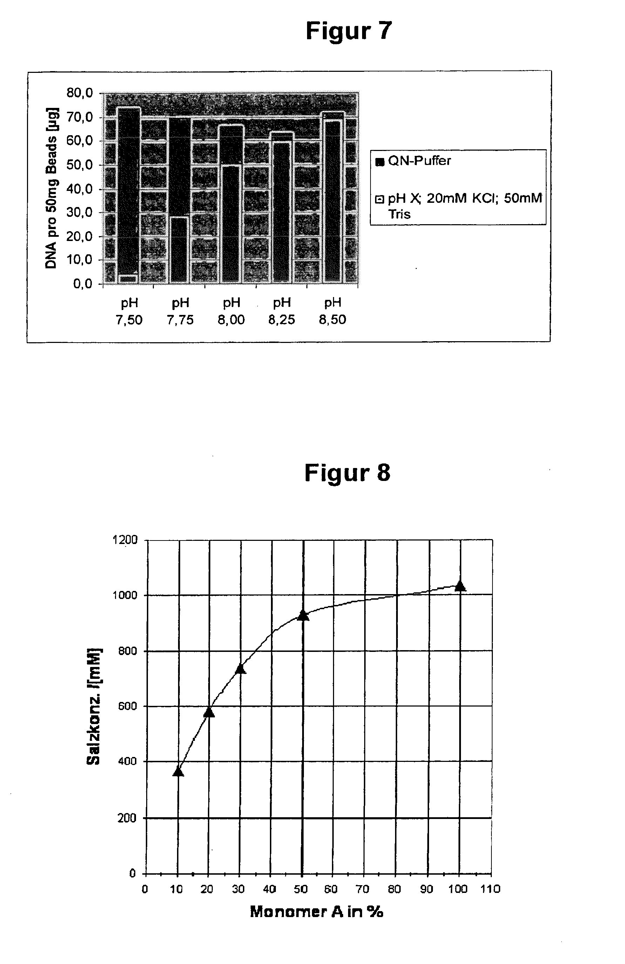

Die Ergebnisse sind in **Fig. 7** gezeigt.

### B) Modifizierung eines Trägermaterials mit Initiatormolekülen

Zur Herstellung der erfindungsgemäßen Random- und/oder Block(-Co)-Polymere werden zuerst Initiatorgruppen T auf das entsprechende Trägermaterial - wie nachfolgend beispielhaft dargestellt - aufgebracht. Diese dienen dann in einem nachfolgenden Schritt als Ausgangspunkt für die Aufbringung von Liganden/Polymerketten aus den Gruppen A und/oder den Gruppen N.

### B.1) Silanisierung von [3-(2-Bromoisobutyryl)propyl]-ethoxydimethylsilan auf einem Silikagel

### Materialien

Trägermaterial: Silikagel (Fuji MB 1500-40/75 / Lot: HT70594)
Beschichtungsreagenzien: [3-(2-Bromoisobutyryl)propyl]-ethoxydimethylsilan (BPDS).
Silanmenge: 800µmol/ g Trägermaterial

### Herstellungsvorschrift

In einer 250 ml KPG-Rührapparatur (trocken) mit Rückflüsskühler und Wasserabscheider wurden 10,0 g Trägermaterial, 200 ml trockenes Cyclohexan und 2490 mg [3-(2-Bromoisobutyryl)propyl]-ethoxydimethylsilan vorgelegt.

Das Gemisch reagierte unter Rühren (100 1/min) bei 85°C (Ölbadtemperatur) über Nacht (16h). Danach wurde das Ölbad entfernt und die Suspension kühlte innerhalb ½h unter Rühren ab. Das modifizierte Trägermaterial / der Kieseigelinitiator **(KI04)** wurde über eine P3 Fritte abgetrennt und auf dieser mit 7 x 20 mL Hexan gewaschen. Danach wurde das modifizierte Trägermaterial bei 40°C über Nacht (ca. 12h) im Vakuumtrockenschrank getrocknet.

Gemäß der vorstehenden Herstellvorschrift wurden fünf Chargen an Kieselgelinitiatoren **(KI 04 a - e)** hergestellt, die im nachfolgenden Schritt als Ausgangspunkt für die Herstellung der erfindungsgemäßen, schwachen Ionenaustauscher dienten.

### C) Herstellung schwacher Ionenaustauscher mittels ATRP

Zur Herstellung der erfindungsgemäßen, schwachen Ionenaustauscher wurden auf das Ausgangsmaterial, dem Initiatorgruppen T tragenden, modifizierten Trägermaterial (KI04), Randompolymerketten aus den Gruppen A und/oder den Gruppen N aufwachsen gelassen.

### C.1) Aufsetzen von Randompolymeren aus den Gruppen A/N auf die unter B.1 beschriebenen modifizierten Trägermaterialien / Kieselgelinitiatoren

Das Ausgangsmaterial für diese Versuchsreihe waren die Kieselgelinitiatoren **(KI04d)** mit BPDS als Initiator.

Hierauf wurden die Monomere DMAEMA (2-(Dimethylamino)ethylmethacylat / Gruppe A) und HEMA (Hydroxyethylmethacrylat / Gruppe N) im folgenden Verhältnis zueinander aufgebracht:

### DMAEMA:HEMA

| | | | | |
|---|---|---|---|---|
| **100 : 0** | ⇒ | 16,0 mmol : | 0,0 mmol | (KI04d-01) |
| **50 : 50** | ⇒ | 8,0 mmol : | 8,0 mmol | (KI04d-03) |
| **30 : 70** | ⇒ | 4,8 mmol : | 11,2 mmol | (KI04d-04) |
| **20 : 80** | ⇒ | 3,2 mmol : | 12,8 mmol | (KI04d-05) |
| **10 : 90** | ⇒ | 1,6 mmol : | 14,4 mmol | (KI04d-07) |

### Materialien

Kieselgelinitiator: KI04d
Monomere: DMAEMA und HEMA
Ligand: 0,96 mmol 2,2'-Bipyridyl (bdy)
Kupfersalz: 0,48 mmol Kupfer-I-bromid (gereinigt)
Lösemittel: 36 ml Dimethylformamid (DMF)

Verhältnis Initiator: Cu(I) : Ligand = 1 : 4 : 8
Verhältnis Initiator: Monomer(en) = 1 : 133

### Herstellungsvorschrift

In einer 100 ml KPG-Dreihalsrührapparatur (Glasführungshülse) mit Argonanschluss und Blasenzähler (bzw. Anschluss an Membranpumpe) wurden 150 mg bdy und 68,8 mg Kupfer-I-bromid vorgelegt.

Die Apparatur wurde mittels Membranpumpe 1 min lang evakuiert und anschließend mit Argon gefüllt. Diese Prozedur wurde 3 x durchgeführt. Im Anschluss wurde permanent ein leichter Argonstrom in die Apparatur geleitet, um zu verhindern, dass Luftsauerstoff eindringt.

Parallel wurden 36ml DMF (Ligand) und die Monomere entgast.

Dann wurden die Flüssigkeiten in einem Zweihalskolben mit Hahn vorgelegt, wobei die Monomere je nach Herstellung die folgenden Einwaageverhältnisse hatten:
KI04d-01 = 2700 µl DMAEMA : 0,0 µl HEMA
KI04d-03 = 1350 µl DMAEMA : 972 µl HEMA
KI04d-04 = 810 µl DMAEMA : 1360 µl HEMA
KI04d-05 = 540 µl DMAEMA : 1555 µl HEMA
KI04d-07 = 270 µl DMAEMA : 1750 µl HEMA

Die Mischung aus Ligand und Monomer(en) wurde zuerst 5 min im Ultraschallbad behandelt und anschließend wurde mittels Hochvakuum für 5 min der Gasraum evakuiert.

Das Ligand-Monomer-Gemisch wurde zum vorgelegten bdy / Kupfer-I-bromid pipettiert. Die Apparatur wurde mittels Membranpumpe 1 min lang evakuiert und anschließend mit Argon gefüllt. Diese Prozedur wurde 3 x durchgeführt. Im Anschluss wurde permanent ein leichter Argonstrom in die Apparatur geleitet.

Unter rühren (200 1/min) wurde der Kupfer-Ligand-Komplex innerhalb von 15 min gelöst. Danach wurden 1,2 g des Kieselgelinitiators **KI 04d** zugegeben.

Die Apparatur wurde mittels Membranpumpe 1 min lang evakuiert und anschließend mit Argon gefüllt. Diese Prozedur wurde 3 x durchgeführt. Im Anschluss wurde permanent ein leichter Argonstrom in die Apparatur geleitet.

Die Mischung wurde 4h bei 40°C (Ölbadtemperatur) gerührt (200 1/min).

Das Trägermaterial mit den aufgewachsenen Gruppen (im Weiteren kurz Kieselgel genannt) wurde direkt über eine P3 Fritte abgetrennt und nachfolgend hintereinander mit
5 x 8 ml DMF
5 x 8 ml THF
5 x 8 ml 0,1 M EDTA / Acetat - Puffer pH 3,6 (0,2M Na-Acetat mit Eisessig)
2 x 8 ml Tris / NaCl - Puffer pH 7,0
3 x 8 ml Wasser sowie
3 x 8 ml Methanol gewaschen.

Danach wurde das Kieselgel bei 40°C im Vakuumtrockenschrank bis zur Gewichtskonstanz getrocknet.

### Ausbeute:

KI04d-01 = 1,31 g
KI04d-03 = 1,20 g
KI04d-04 = 1,23 g
KI04d-05 = 1,29 g
KI04d-07 = 1,21 g

Die resultierenden Anionentauscher (das Modifizierte Trägermaterial resp. Kieselgel) und das Ausgangsmaterial (der Kieselgelinitiator) wurden - wie nachfolgend näher erläutert - im Hinblick auf ihre pDNA Bindekapazität untersucht und es wurden entsprechende Elutionsprofile aufgenommen.

Ferner wurden die Anionenaustauscher auf den CHN-Gehalt untersucht und daraus die Polymerkettenlängen und das Verhältnis A zu N bestimmt. Es konnte gezeigt werden, dass die eingesetzten (Mol-)Mengenverhältnisse der Monomere sich im resultierenden Kieselgel widerspiegeln.

### Aufreinigung von Plasmid DNA mit dem modifizierten Trägermaterial KI04d-01 bis KI04d-07 unter Bestimmung des Elutionspunktes

| Eingesetzte Puffer: | | | | | |
|---|---|---|---|---|---|
| | pH-Wert | NaCl | | Na-Acetat-Trihydrat | |
| | | [g/L] | [mmol/L] | [g/L] | [mmol] |
| Bindepuffer / Waschpuffer | 5 | 0 | 0 | 6,8 | 50 |
| | | | | | |
| | pH-Wert | KCl | | Tris | |
| | | [g/L] | [mmol/L] | [g/L] | [mmol] |
| Elutionspuffer | 8,5 | 1,49 | 20 | 6,07 | 50 |
| | | | | | |
| | pH-Wert | NaCl | | Tris | |
| | | [g/L] | [mmol/L] | [g/L] | [mmol] |
| Tris-NaCl-Puffer | 10 | 116,88 | 2000 | 6,06 | 50 |

### Durchführungsvorschrift

Es wurden Einfachbestimmungen pro Kieselgel und für jeden der zwei Elutionspuffer durchgeführt.

Hierfür wurden je 50,00 mg (+/- 5 mg) Kieselgel in ein 2 ml Safe Lock ReaktionsTube der Firma Eppendorf (im Folgenden Eppendorf-Tube genannt) eingewogen und für eine DNA-Lösung wurden pro Kieselgeleinwaage je 100 µg pcmvb mit dem Bindepuffer auf 1 ml aufgefüllt.

Danach wurden in die Eppendorf-Tubes mit den Kieselgeleinwaagen je 1 ml der DNA-Lösung gegeben, die Eppendorf-Tubes verschlossen und kurz gevortext.

Dann wurden die Eppendorf-Tubes bei mittlerer Geschwindigkeit für 5 min in den End-Over-End-Schüttler eingespannt. Nach dem Schütteln wurden die Eppendorf-Tubes kurz zentrifugiert.

Anschließend wurde die Kieselgel-DNA-Suspension mit einer gekürzten Spitze in eine vorbereitete Tip 20-Form mit unterer Fritte (im Folgenden Tips genannt), unter welcher ein 2 ml Eppendorf-Tube zum Auffangen des Überstandes bereitgestellt wurde, pipettiert.

Um evtl. Materialreste in den Eppendorf-Tubes zu erfassen, wurden diese mit je 0,9 ml Bindepuffer ausgewaschen, der ebenfalls auf die entsprechenden Tips gegeben wurde.

Nachdem keine Flüssigkeit mehr durchtropfte wurde die restliche Flüssigkeit vollständig durchgedrückt und neue Auffang-Eppendorf-Tubes unter die Tip-Säulen gestellt.

Danach wurde zum Waschen je 1 ml Waschpuffer in die Tips pipettiert. Sobald keine Flüssigkeit mehr durchtropfte wurde die restliche Flüssigkeit vollständig durchgedrückt, das Auffang-Eppendorf-Tube (mit der Waschlösung) auf die Seite gestellt und neue Auffang-Eppendorf-Tubes unter die Tip-Säulen gestellt.

Danach wurde je 1 ml des entsprechenden Elutionspuffers in die Tips pipettiert. Sobald auch hier keine Flüssigkeit mehr durchtropfte wurde die restliche Flüssigkeit vollständig durchgedrückt, das Auffang-Eppendorf-Tube (mit dem Eluat) auf die Seite gestellt und neue Auffang-Eppendorf-Tubes unter die Tip-Säulen gestellt.

Zum Lösen der restlichen gebundenen DNA wurden je 1 ml Tris-NaCl-Puffer in die Tips pipettiert. Sobald keine Flüssigkeit mehr durchtropfte wurde die restliche Flüssigkeit vollständig durchgedrückt, das Auffang-Eppendorf-Tube (mit der Tris-NaCl-Lösung) auf die Seite gestellt und die Tip-Säulen verworfen.

Zur Messung der DNA-Konzentration mittels SpectraMax wurden je 100 µl der abgenommenen Lösungen in einer UV-Platte überführt.

Als Blank für den Überstand wurden 10 µl EB-Puffer und 180 µl Bindepuffer angesetzt. Von dieser Lösung wurden 100 µl in die UV-Platte gegeben. Als Referenz zur eingesetzten DNA-Menge wurden 100 µl DNA-Lösung mit 90 µl Bindepuffer angesetzt und 100µL von dieser Lösung in die UV-Platte gegeben.

Als Blanks für die Eluate wurden 100 µl des entsprechenden Elutionspuffers verwendet.

Als Blank für die Tris-NaCl-Lösung wurden 100µl Tris-NaCl-Puffer verwendet.

Die NaCl-Konzentration, bei der erstmalig signifikante Mengen an DNA eluiert wurde, wurde als Elutionspunkt festgehalten.

| | Monomere in mmol | | | | | Total pDNA / |
|---|---|---|---|---|---|---|
| Kieselgele | A | N | A+N | A in % | Elutionspunkte | [µg / 50 mg] |
| KI 04d-01 | 16 | 0 | 16 | 100 | 1044 | 85 |
| KI 04d-03 | 8 | 8 | 16 | 50 | 928 | 23 |
| KI 04d-04 | 4,8 | 11,2 | 16 | 30 | 738 | 20 |
| KI 04d-05 | 3,2 | 12,8 | 16 | 20 | 582 | 31 |
| KI 04d-07 | 1,6 | 14,4 | 16 | 10 | 370 | 10 |

Dabei zeigten auch hier die (vorstehend in der Tabelle aufgeführten sowie in **Fig. 8** dargestellten) Ergebnisse, dass die Einführung der Gruppen N den Elutionspunkt signifikant senkt.

Das ausschließlich mit den Gruppen A (DMAEMA) beschichtete Trägermaterial / Kieselgel (KI04d-01) zeigte bei einem pH-Wert von 8,5 den Elutionspunkt bei 1032 mM NaCl. Es wurden also recht hohe Salzkonzentrationen benötigt, um die Nukleinsäuren zu eluieren. Für das gemischt A/N-modifizierte Kieselgel KI04d-05 ergab sich der Elutionspunkt bei ∼ 580 mM NaCl. Die zur Elution notwendige Ionenstärke wurde somit um fast 50% reduziert.

### C.2) Aufsetzen von Blockpolymeren aus den Gruppen A/N auf die unter B.1 beschriebenen modifizierten Trägermaterialien / Kieselgelinitiatoren

Das Ausgangsmaterial für diese Versuchsreihe waren die Kieselgelinitiatoren **(KI04e)** mit BPDS als Initiator.

Hierauf wurde in einem ersten Schritt eine Aminopolymerkette aus zumindest einem Monomer der Gruppe A aufgesetzt. Und danach wurde in einem zweiten Schritt auf das so vorbereitete Kieselgel eine Polymerkette aus zumindest einem Monomer der Gruppe N (vorzugsweise HEMA) aufgebracht.

### C.2.1) Schritt 1

### Materialien

Kieselgelinitiator: KI04e
Monomer: DMAEMA
Ligand: 7,2 mmol 2,2'-Bipyridyl (bdy)
Kupfersalz: 3,6 mmol Kupfer-I-bromid (gereinigt)
Lösemittel: 270 ml Dimethylformamid (DMF)

Verhältnis Initiator: Cu(I) : Ligand = 1 : 4 : 8
Verhältnis Initiator: Monomer = 1 : 133

### Herstellungsvorschrift

In einer 500 ml KPG-Dreihalskolben-Rührapparatur (Glasführungshülse) mit Argonanschluss und Blasenzähler (bzw. Anschluss an Membranpumpe) wurden 1125 mg bdy und 516 mg Kupfer-I-bromid vorgelegt.

Die Apparatur wurde mittels Membranpumpe 1 min lang evakuiert und anschließend mit Argon gefüllt. Diese Prozedur wurde 3 x durchgeführt. Im Anschluss wurde permanent ein leichter Argonstrom in die Apparatur geleitet, um zu verhindern, dass Luftsauerstoff eindringt.

Parallel wurden 270 ml DMF und 20,25 ml DMAEMA entgast. Hierzu wurden die beiden Flüssigkeiten in einem Zweihalskolben mit Hahn vorgelegt. Die Mischung wurde zuerst 5 min im Ultraschallbad behandelt und anschließend wurde mittels Hochvakuum 5 min der Gasraum evakuiert.

Das DMF-DMAEMA-Gemisch wurde zum bdy / Kupfer-I-bromid pipettiert.

Die Apparatur wurde mittels Membranpumpe 1 min lang evakuiert und anschließend mit Argon gefüllt. Diese Prozedur wurde 3 x durchgeführt. Im Anschluss wurde permanent ein leichter Argonstrom in die Apparatur geleitet.

Unter Rühren (200 1/min) wurde der Kupfer-Ligand-Komplex innerhalb von 15 min gelöst. Danach wurden 9 g des Kieselgelinitiators **KI04e** zugegeben.

Die Apparatur wurde mittels Membranpumpe 1 min lang evakuiert und anschließend mit Argon gefüllt. Diese Prozedur wurde 3 x durchgeführt. Im Anschluss wurde permanent ein leichter Argonstrom in die Apparatur geleitet.

Die Mischung wurde 4h bei 40°C (Ölbadtemperatur) gerührt (200 1/min).

Das Kieselgel **KI04e-01** mit den aufgewachsenen Gruppen A wurde direkt über eine P3 Fritte abgetrennt und nachfolgend hintereinander mit
5 x 60 ml DMF
5 x 60 ml THF
5 x 60 ml 0,1 M EDTA / Acetat - Puffer pH 3,6 (0,2M Na-Acetat mit Eisessig)
2 x 60 ml Tris / NaCl - Puffer pH 7,0 sowie
3 x 60 ml Wasser gewaschen.

Danach wurde das Kieselgel **KI04e-01** bei 40°C im Vakuumtrockenschrank bis zur Gewichtskonstanz getrocknet.

### C.2.2) Schritt 2

### Materialien

Kieselgelinitiator: KI04e-01
Monomer: HEMA
Ligand: 0,96 mmol 2,2'-Bipyridyl (bdy)
Kupfersalz: 0,48 mmol Kupfer-I-bromid (gereinigt)
Lösemittel: 36 ml Dimethylformamid (DMF)

Verhältnis Initiator: Cu(I) : Ligand = 1 : 4 : 8
Verhältnis Initiator: Monomer = 1 : 200

### Herstellungsvorschrift

In einer 100 ml KPG-Dreihalskolben-Rührapparatur (Glasführungshülse) mit Argonanschluss und Blasenzähler (bzw. Anschluss an Membranpumpe) wurden 150 mg bdy und 68,8 mg Kupfer-I-bromid vorgelegt.

Die Apparatur wurde mittels Membranpumpe 1 min lang evakuiert und anschließend mit Argon gefüllt. Diese Prozedur wurde 3 x durchgeführt. Im Anschluss wurde permanent ein leichter Argonstrom in die Apparatur geleitet, um zu verhindern, dass Luftsauerstoff eindringt.

Parallel wurden 36 ml DMF und 2916 µl HEMA entgast. Hierzu wurden die beiden Flüssigkeiten in einem Zweihalskolben mit Hahn vorgelegt. Die Mischung wurde zuerst 5 min im Ultraschallbad behandelt und anschließend wurde mittels Hochvakuum 5 min der Gasraum evakuiert.

Das DMF-HEMA-Gemisch wurde zum bdy / Kupfer-I-bromid pipettiert.

Die Apparatur wurde mittels Membranpumpe 1 min lang evakuiert und anschließend mit Argon gefüllt. Diese Prozedur wurde 3 x durchgeführt. Im Anschluss wurde permanent ein leichter Argonstrom in die Apparatur geleitet.

Unter Rühren (200 1/min) wurde der Kupfer-Ligand-Komplex innerhalb von 15 min gelöst. Danach wurden 1,2 g des Kieselgelinitiators **KI04e-01** zugegeben.

Die Apparatur wurde mittels Membranpumpe 1 min lang evakuiert und anschließend mit Argon gefüllt. Diese Prozedur wurde 3 x durchgeführt. Im Anschluss wurde permanent ein leichter Argonstrom in die Apparatur geleitet.

Die Mischung wurde 6h bei 40°C (Ölbadtemperatur) gerührt (200 1/min).

Das Kieselgel **KI04e-01-01** mit den aufgewachsenen Gruppen A wurde direkt über eine P3 Fritte abgetrennt und nachfolgend hintereinander mit
5 x 8 ml DMF
5 x 8 ml THF
5 x 8 ml 0,1 M EDTA / Acetat - Puffer pH 3,6 (0,2M Na-Acetat mit Eisessig)
2 x 8 ml Tris / NaCl - Puffer pH 7,0
3 x 8 ml Wasser sowie
3 x 8 ml Methanol gewaschen.

Danach wurde das Kieselgel **KI04e-01-01** bei 40°C im Vakuumtrockenschrank bis zur Gewichtskonstanz getrocknet.

Durch die mit diesem Kieselgel durchgeführten Elutionsversuche konnte gezeigt werden, dass auch die aufgesetzte Polymerkette aus Monomeren der Gruppe N deutlich zur sterischen Abschirmung der Nukleinsäure(n) beitrug und damit die Bindestärke der Nukleinsäure(n) signifikant herabgesetzt wurde.

## Patentansprüche

1. Verfahren zur Aufreinigung von Nukleinsäuren mit einer nukleinsäurebindenden Phase durch Bindung und Elution, **dadurch gekennzeichnet, dass** die nukleinsäurebindende Phase aufweist:
(i) nukleinsäurebindende Gruppen A, wobei diese einen pK-Wert von 8 bis 13 aufweisen;
(ii) ladungsneutrale Gruppen N, wobei diese bei dem eingesetzten Bindungs-pH Wert und beim eingesetzten Elutions-pH Wert neutral geladen sind;
wobei die nukleinsäurebindende Phase ein Trägermaterial aufweist, das mit unterschiedlichen Liganden (Liganden I und Liganden II) versehen ist, wobei die Liganden I wenigstens eine Gruppe A und die Liganden II wenigstens eine Gruppe N aufweisen;
und das Verfahren wenigstens die nachfolgenden Schritte aufweist:
(a) Bindung der Nukleinsäuren an die nukleinsäurebindende Phase bei einem pH-Wert, der unterhalb des pK - Wertes der nukleinsäurebindenden Gruppen A liegt (Bindungs- pH Wert);
(b) Elution der Nukleinsäuren bei einem pH-Wert, der oberhalb des Bindungs-pH Wertes liegt (Elutions-pH Wert).

2. Verfahren nach Anspruch 1, **dadurch gekennzeichnet, dass** die nukleinsäurebindende Phase ein modifiziertes Trägermaterial ist, wobei die Modifikation ausgewählt ist aus der Gruppe:
(i) Modifikation des Trägermaterials mit einer wenigstens die Liganden I und II aufweisenden Mischung, wobei die Liganden I wenigstens eine Gruppe A aufweisen und die Liganden II wenigstens eine Gruppe N aufweisen;
(ii) Modifikation des Trägermaterials mit Gruppen A und N, wobei die Gruppen A sterisch durch Verbindungen abgeschirmt werden, die wenigstens eine Gruppe N aufweisen;
(iii) Modifikation des Trägermaterials durch eine Kombination aus den Ausführungsformen (i) und (ii).

3. Verfahren nach Anspruch 1 oder 2, **dadurch gekennzeichnet, dass** die nukleinsäurebindende Phase ein Trägermaterial aufweist, das eines oder mehrere der folgenden Merkmale erfüllt:
(i) das Trägermaterial ist ein oxidisches Material; und/oder
(ii) das Trägermaterial ist ausgewählt aus der Gruppe von Al₂O₃, TiO₂, ZrO₂, Ta₂O₅, SiO₂ und Polykieselsäure; und/oder
(iii) das Trägermaterial ist SiO₂ oder Polykieselsäure; und/oder
(iv) dasTrägermaterial ist ein organisches Polymer, insbesondere ausgewählt aus der Gruppe Polystyrol und seine Derivate, Polyacrylat und -methacrylat, und seine Derivate, Polyurethan, Nylon, Polyethylen, Polypropylen, Polybutyliden und Copolymere aus diesen Materialien; und/oder
(v) das Trägermaterial ist ein Polysaccharid, insbesondere ein Hydrogel wie Agarose, Cellulose, Dextran, Sephadex, Sephacryl oder Chitosan; und/oder
(vi) das Trägermaterial ist Glas oder Metall; und/oder
(vii)das Trägermaterial ist magnetisch.

4. Verfahren nach einem oder mehreren der Ansprüche 1 bis 3, **dadurch gekennzeichnet, dass** die nukleinsäurebindende Phase Gruppen A aufweist, die eines oder mehrere der nachfolgenden Merkmale erfüllen:
(i) die Gruppen A weisen einen pK-Wert von 9 bis 13, vorzugsweise 10 bis 12, besonders bevorzugt 10 bis 11 auf; und/oder
(ii) die Gruppen A sind tertiäre, sekundäre oder primäre Aminogruppen; und/oder
(iii) die Gruppen A sind Bestandteil von polymerisationsfähigen Monomeren; und/oder
(iv) die Gruppen A sind an ein als Reaktivsilan vorab aufgebrachtes Initiatormolekül gebunden; und/oder
(v) die Gruppen A wurden als Bestandteil von reaktiven Silangruppen eingeführt; und/oder
(vi) die Gruppen A wurden als Bestandteil von Verbindungen eingeführt, die ausgewählt sind aus der Gruppe bestehend aus: 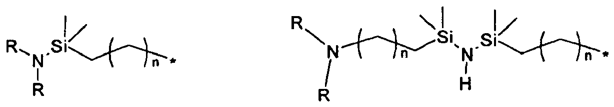 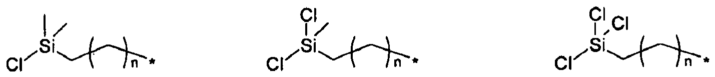 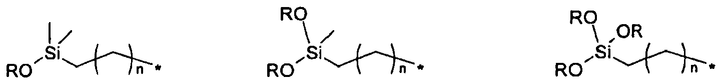 Wobei
n: 1 bis 5
R eine C1 bis C6, vorzugsweise eine C1 bis C3 Alkylgruppe ist; und
* Amino-, Aminomethyl-, Aminoethyl-, Aminopropyl-, Dimethylamino-, Diethylamino-, Diisopropylamino-, Dipropylamino-, Diethanolamino-, Dipropanolamino-, Diethylentriamin-, Triethylentetramin-, Tetraethylenpentamin, Etheramin, Polyetheramin, 4-Diisobutylamino-I-butan, 6-Dipropylamino-I-hexan; und/oder
(vii) die Gruppen A wurden als Bestandteil von Verbindungen eingeführt, die ausgewählt sind aus der Gruppe bestehend aus Diethylaminopropyltrimethoxysilan, Dimethylamino-propyltrimethoxysilan und N,N-Diisopropylaminopropyltrimethoxysilan.

5. Verfahren nach einem oder mehreren der Ansprüche 1 bis 4, **dadurch gekennzeichnet, dass** der Bindungsschritt (a) und/oder der Elutionsschritt (b) unter Bedingungen erfolgt, die eines oder mehrere der nachfolgenden Merkmale erfüllen:
(i) im Elutionsschritt (b) liegt der Elutions-pH Wert unterhalb des pK-Wertes der Gruppen A; und/oder
(ii) die Salzkonzentration im Bindungs und/oder Elutionspuffer beträgt 1mM bis 1000mM, 1 mM bis 500mM, 1mM bis 250mM oder 1mM bis 100mM; und/oder
(iii) die Salzkonzentration ist im Bindungsschritt (a) und/oder im Elutionsschritt (b) unverändert oder wird bei der Elution angehoben; und/oder
(iv) der pH-Wert im Bindungspuffer beträgt pH 2 bis pH 8, pH 2 bis pH 7,5, pH 4 bis pH 8 oder pH 4 bis pH 7,5 und/oder
(v) der pH-Wert im Elutionspuffer beträgt pH 2 bis pH 10, pH 4 bis pH 10, pH 7 bis pH 10 oder pH 8 bis pH 9; und/oder
(vi) die Temperatur ist im Bindungsschritt (a) und/oder im Elutionsschritt (b) unverändert oder wird bei der Elution angehoben; und/oder
(vii) die Temperatur im Elutionsschritt beträgt 2 °C bis 95 °C oder 21 °C bis 60 °C

6. Verfahren nach einem oder mehreren der Ansprüche 1 bis 5, **dadurch gekennzeichnet, dass** die Gruppen N eines oder mehrere der nachfolgenden Merkmale erfüllen:
(i) die Gruppen N sind ausgewählt aus der Gruppe bestehend aus Hydroxylgruppen, Diolgruppen, Triolgruppen, Polyolgruppen, Saccharide, Epoxidgruppen, Halogenide, Alkylgruppen, vorzugsweise C1-C6 Alkylgruppen, Alken-, Alkylengruppen, Imidgruppen, Ethergruppen, oder Polyethergruppen und/oder
(ii) die Gruppen N sind in den pH Bereichen 2< pH <12 ladungsneutral; und/oder
(iii) die Gruppen N wurden als Bestandteil von polymerisationsfähigen Monomeren eingeführt; und/oder
(iv) die Gruppen N liegen an ein als Reaktivsilan vorab aufgebrachtes Initiatormolekül gebunden vor; und/oder
(v) die Gruppen N wurden als Bestandteil von reaktiven Silangruppen eingebracht; und/oder
(vi) die Gruppen N wurden als Bestandteil von Verbindungen eingeführt, die ausgewählt sind aus der Gruppe bestehend aus 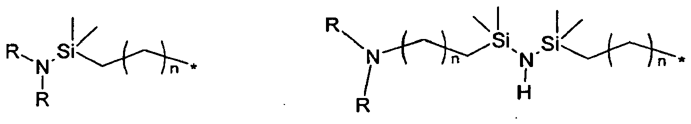 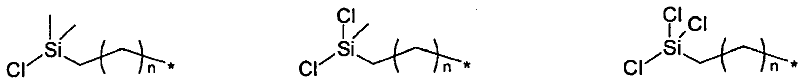 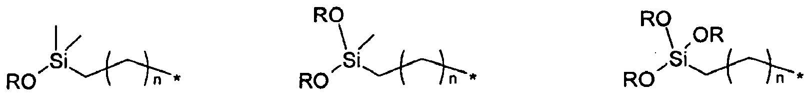 wobei
n= 1-5
R eine C1 bis C6, vorzugsweise C1 bis C3 Alkylgruppe, insbesondere Methyl, Ethyl, Propyl, i-Propyl ist;
* Amino-, Aminomethyl-, Aminoethyl-, Aminopropyl-, Dimethylamino-, Diethylamino-, Diisopropylamino-, Dipropylamino-, Diethanolamino-, Dipropanolamino-, Diethylentriamin-, Triethylentetramin-, Tetraethylenpentamin, Etheramin, Polyetheramin, 4-Diisobutylamino-I-butan, 6-Dipropylamino-I-hexan, Hydroxymethyl-, Hydroxyethyl-, Hydroxypropyl-, Ethandiol-, Propandiol-, Propantriol-, Butantriol, 3-Glycidoxypropyl, Ethylglycidylether, Alkylrest, insbesondere ein C1 bis C4 Alkylrest, insbesondere Methyl, Ethyl, Propyl, i-Propyl, Butyl, i-Butyl, Halogenid oder Wasserstoff ist.

7. Verfahren nach einem oder mehreren der Ansprüche 1 bis 6, **dadurch gekennzeichnet, dass** eines oder mehrere der folgenden Merkmale erfüllt wird:
(i) das Trägermaterial wurde durch Silanisierung mit den Gruppen A und N versehen; und/oder
(ii) die Gruppen A und/oder N und/oder T wurden über Reaktivsilane eingebracht; und/oder
(iii) die Gruppen A und/oder N wurden durch monofunktionale, bi- oder trifunktionale Reaktivsilane oder einem Gemisch aus mindestens zwei unterschiedlich funktionalen Reaktivsilanen eingebracht; und/oder
(iv) Die Gruppen A und/oder N wurden durch Reaktivsilane eingebracht, die ausgewählt sind aus der Gruppe von Aminosilane, Disilazanen, Chlorsilanen oder Alkoxisilanen; und/oder
(v) Der Anteil der Gruppen A bezogen auf die Gruppen N beträgt 1% bis 99%, 1 bis 50%, vorzugsweise 1% bis 25%.

8. Verfahren nach einem oder mehreren der Ansprüche 2 bis 7, **gekennzeichnet durch** eines oder mehrere der nachfolgenden Merkmale:
(i) das Trägermaterial wurde mit Initiatormolekülen T modifiziert, wobei im Anschluss die Gruppen A und/oder Gruppen N in Form von Monomeren eingebracht wurden; und/oder
(ii) das Trägermaterial wurde mit Initiatormolekülen T modifiziert, wobei die Initiatormoleküle eines oder mehrere der folgenden Merkmale aufweisen:
(aa) die Initiatormoleküle sind reaktive Silane; und/oder
(bb) die Initiatormoleküle sind ausgewählt aus der Gruppe bestehend aus 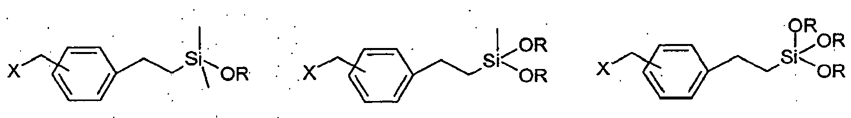 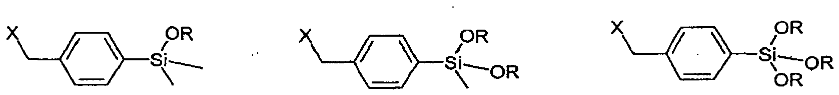 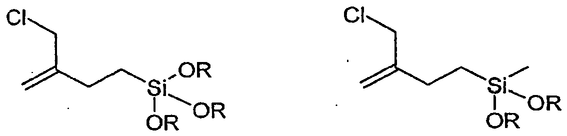 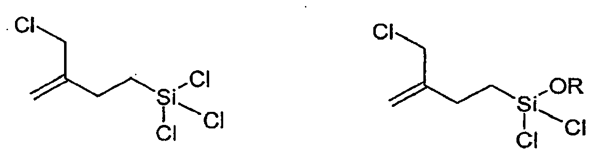 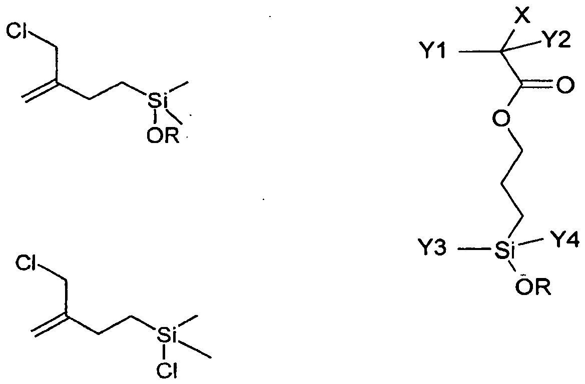
Wobei X = Halogen, insbesondere Cl, Br, I
Y1, Y2, Y3 oder Y4 = unabhängig voneinander R, OR, OH oder H und R = C1 - C3 Alkyl ist.
(iii) das Initiatormolekül ist 2-(Chloromethyl)allyltrimethoxysilan;
(iv) das Initiatormolekül ist [3-(2-Bromoisobutyryl)propyl]-ethoxydimethylsilan
(v) die Gruppen A wurden **durch** Monomere eingebracht, die ausgewählt sind aus der Gruppe
N-(3-Aminomethyl)methacrylamid, N-(3-Aminoethyl)methacrylamid,
N-(3-Aminopropyl)methacrylamid,N-(3-Aminoisopropyl)methacrylamid
N,N-Dimethylacrylamid, N,N-Diethylacrylamid, N,N-Diisopropylacrylamid
N,N-(Dimethylamino)ethylacrylamid, N,N-(Dimethylamino)ethylacrylat,
N,N-(Dimethylamino)ethylmethacrylamid, N,N-(Dimethylamino)ethylmethacrylat,
N,N-(Dimethylamino)propylacrylamide,N,N-(Dimethylamino)propylacrylat,
N,N-(Dimethylamino)propylmethacrylamide,N,N-(Dimethylamino)propylmethacrylat,
N,N-(Diethylamino)ethylacrylamid, N,N-(Diethylamino)ethylacrylat,
N,N-(Diethylamino)ethylmethacrylamid, N,N-(Diethylamino)ethylmethacrylat,
N,N-(Diethylamino)propylacrylamide,N,N-(Diethylamino)propylacrylat,
N,N-(Diethylamino)propylmethacrylamide,N,N-(Diethylamino)propylmethacrylat,
N,N-(Diisopropylamino)ethylacrylamid, N,N-(Diisopropylamino)ethylacrylat,
N,N-(Diisopropylamino)ethylmethacrylamid, N,N-(Diisopropylamino)ethylmethacrylat,
N,N-(Diisopropylamino)propylacrylamide,N,N-(Diisopropylamino)propylacrylat,
N,N-(Dimethylamino)propylmethacrylamide,N,N-(Dimethylamino)propylmethacrylat, 2-(Diisopropylamino)ethylmethacrylat
und/oder
(vi) die Gruppen N wurden **durch** Monomere eingebracht, die ausgewählt sind aus der Gruppe Butylacrylat, Propylacrylat, Ethylacrylat, Methylacrylat, Glycidylmethacrylat, Hydroxyethylmethacrylat (HEMA), Glycidoxypropylmethacrylat, Glycerolmonomethacrylat (Isomerengemisch), Glycolmonomethacrylat, Glycidylacrylat, Hydroxyethylacrylat, Glycidoxypropylacrylat, Glycerolmonoacrylat (Isomerengemisch), Glycolmonoacrylat und N-Acryloxysuccinimid.

9. Verfahren nach einem oder mehreren der Ansprüche 1 bis 8, **dadurch gekennzeichnet, dass** das Trägermaterial Initiatorgruppen T aufweist, die zumindest teilweise mit Verbindungen funktionalisiert sind, die Gruppen A aufweisen, wobei die Gruppen A durch Gruppen N sterisch abgeschirmt sind, wobei die sterische Abschirmung durch eine oder mehrere der folgenden Modifikationen erzeugt wird:
(i) die Gruppen N liegen in Form von kettenförmigen verzweigten oder unverzweigten Oligomeren oder Polymeren vor, die die Gruppen A sterisch abschirmen; und/oder
(ii) die Gruppen N liegen in Form von aus Monomeren gebildeten Oligomeren oder Polymeren vor, wobei diese eine Kettenlänge mit n= 10 - 1000, bevorzugt n = 10 - 100 aufweisen; und/oder
(iii) Die Gruppen N liegen als Polyacrylat, vorzugsweise gemäß der der folgenden Formel vor: 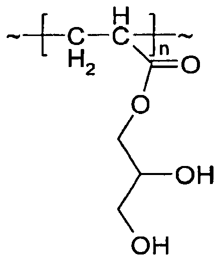
(iv) die Gruppen N besitzen bzw. sind räumlich ausgedehnte Gruppen wie z.B. Isopropyl, iso-Butyl, tert.-Butyl oder n-Hexylreste.

10. Verfahren zur Herstellung eines nukleinsäurebindenden Trägermaterials wie in einem oder mehreren der Ansprüche 1 bis 4 sowie 6 bis 9 definiert, wobei das Verfahren nach wenigstens einer der nachfolgenden Alternativen (A) bis (D) durchgeführt wird:
Alternative (A): Funktionalisierung des Trägermaterials mit einer wenigstens die Liganden I und II aufweisenden Mischung, wobei die Liganden I wenigstens eine Gruppe A aufweisen und die Liganden II wenigstens eine Gruppe N aufweisen; und/oder
Alternative (B): (a) Funktionalisierung des Trägermaterials mit einer Mischung aus Initiatormolekülen T und Liganden, die wenigstens eine Gruppe A aufweisen (b) Zugabe von wenigstens eine Gruppe N aufweisenden Verbindungen und Anbindung dieser an die Initiatormoleküle T; und/oder
Alternative (C): Funktionalisierung des Trägermaterials mit Gruppen A und N, wobei die Gruppen A sterisch durch Verbindungen abgeschirmt werden, die wenigstens eine Gruppe N aufweisen; und/oder
Alternative (D): Funktionalisierung des Trägermaterials durch eine Kombination aus wenigstens zwei der Alternativen (A) bis (C).

11. Das Verfahren nach Anspruch 10, **dadurch gekennzeichnet, dass** es wenigstens eines der nachfolgenden Merkmale erfüllt:
(i) die Gruppen A sind tertiäre, sekundäre oder primäre Aminogruppen; und/oder
(ii) die Gruppen N sind ausgewählt aus der Gruppe bestehend aus Hydroxylgruppen, Diolgruppen, Triolgruppen, Polyolgruppen, Saccharide, Epoxidgruppen, Halogenide, C1-C6 Alkylgruppen, Alken-, Alkylengruppen, Imidgruppen, Ethergruppen, oder Polyethergruppen und/oder
(iii) die Gruppen N sind beim Elutions pH-Wert und vorzugsweise in den spezifizierten pH Bereichen 2< pH <12 ladungsneutral; und/oder
(iv) die Gruppen A und/oder N werden durch Monomere eingeführt; und/oder
(v) die Gruppen A und/oder N werden durch Silanisierung eingeführt; und/oder
(vi) die Gruppen A und/oder N werden durch Reaktivsilane eingeführt; und/oder
(vii)zur Einführung der Gruppen A und/oder N werden monofunktionale oder bi- und/oder trifunktionale Reaktivsilane eingesetzt; und/oder
(viii) zur Einführung der Gruppen A und/oder N werden Reaktivsilane eingesetzt, die ausgewählt sind aus der Gruppe von Aminosilanen, Disilazanen, Chlorsilanen oder Alkoxisilanen; und/oder
(ix) die Gruppen A und/oder N werden durch Reaktivsilane eingeführt, die ausgewählt sind aus der Gruppe bestehend aus: 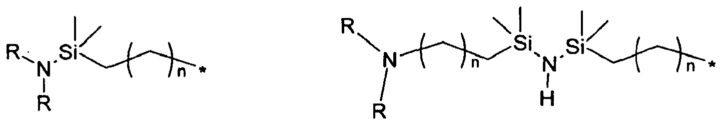 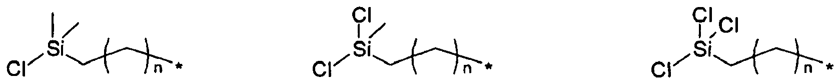 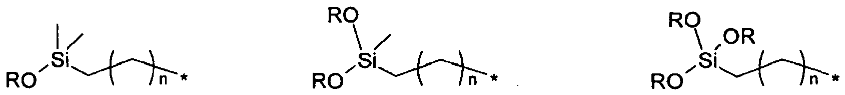 wobei
n= 1-5
R eine C1 bis C6, vorzugsweise C1 bis C3 Alkylgruppe, insbesondere Methyl, Ethyl, Propyl, i-Propyl ist;
* Amino-, Aminomethyl-, Aminoethyl-, Aminopropyl-, Dimethylamino-, Diethylamino-, Diisopropylamino-, Dipropylamino-, Diethanolamino-, Dipropanolamino-, Diethylentriamin-, Triethylentetramin-, Tetraethylenpentamin, Etheramin, Polyetheramin, 4-Diisobutylamino-I-butan, 6-Dipropylamino-I-hexan, Hydroxymethyl-, Hydroxyethyl-, Hydroxypropyl-, Ethandiol-, Propandiol-, Propantriol-, Butantriol, 3-Glycidoxypropyl, Ethylglycidylether, Alkylrest, insbesondere ein C1 bis C4 Alkylrest, insbesondere Methyl, Ethyl, Propyl, i-Propyl, Butyl, i-Butyl, Halogenid oder Wasserstoff ist.
(x) die Gruppen A und N werden in einem Verhältnis eingeführt, so dass der Anteil der A Gruppen 1% bis 99%, 1 bis 50%, vorzugsweise 1% bis 25% beträgt; und/oder
(xi) das Trägermaterial wird mit Initiatormolekülen T modifiziert, die eines oder mehrere der folgenden Merkmale aufweisen:
(aa) die Initiatormoleküle werden durch Silanisierung aufgebracht; und/oder
(bb) die Initiatormoleküle sind Silane; und/oder
(cc) die Initiatormoleküle sind ausgewählt aus der Gruppe bestehend aus 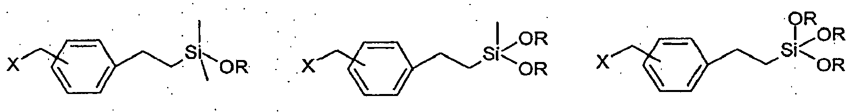 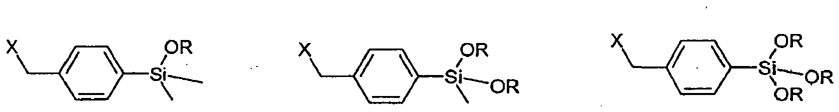 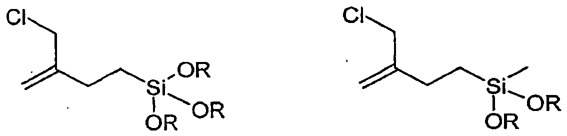 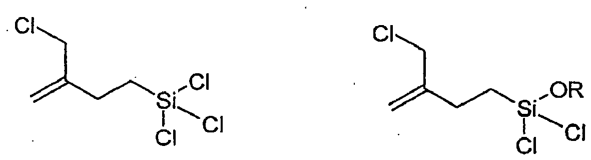 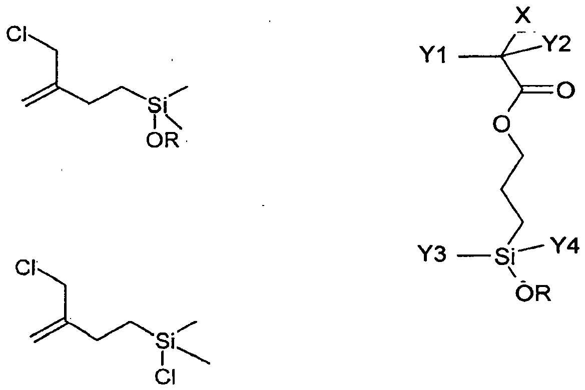
Wobei X = Halogen, insbesondere Cl, Br, I
Y1, Y2, Y3 oder Y4 = unabhängig voneinander R, OR, OH oder H
und R = C1 - C3 Alkyl ist.
(xii) als wird Initiatormolekül 2-(Chloromethyl)allyltrimethoxysilan verwendet;
(xiii) als wird Initiatormolekül [3-(2-Bromoisobutyryl)propyl]-ethoxydimethylsilan verwendet; und/oder
(xiv) die Gruppen A werden durch Monomere eingebracht, die ausgewählt sind aus der Gruppe
2-(Diisopropylamino)ethylmethacrylat
N-(3-Aminomethyl)methacrylamid, N-(3-Aminoethyl)methacrylamid,
N-(3-Aminopropyl)methacrylamid,N-(3-Aminoisopropyl)methacrylamid
N,N-Dimethylacrylamid, N,N-Diethylacrylamid, N,N-Diisopropylacrylamid
N,N-(Dimethylamino)ethylacrylamid, N,N-(Dimethylamino)ethylacrylat,
N,N-(Dimethylamino)ethylmethacrylamid, N,N-(Dimethylamino)ethylmethacrylat,
N,N-(Dimethylamino)propylacrylamide,N,N-(Dimethylamino)propylacrylat,
N,N-(Dimethylamino)propylmethacrylamide,N,N-(Dimethylamino)propylmethacrylat,
N,N-(Diethylamino)ethylacrylamid, N,N-(Diethylamino)ethylacrylat,
N,N-(Diethylamino)ethylmethacrylamid, N,N-(Diethylamino)ethylmethacrylat,
N,N-(Diethylamino)propylacrylamide,N,N-(Diethylamino)propylacrylat,
N,N-(Diethylamino)propylmethacrylamide,N,N-(Diethylamino)propylmethacrylat,
N,N-(Diisopropylamino)ethylacrylamid, N,N-(Diisopropylamino)ethylacrylat,
N,N-(Diisopropylamino)ethylmethacrylamid, N,N-(Diisopropylamino)ethylmethacrylat,
N,N-(Diisopropylamino)propylacrylamide,N,N-(Diisopropylamino)propylacrylat,
N,N-(Dimethylamino)propylmethacrylamide,N,N-(Dimethylamino)propylmethacrylat, und/oder
(xiv) die Gruppen N werden durch Monomere eingebracht, die ausgewählt sind aus der Gruppe Butylacrylat, Propylacrylat, Ethylacrylat, Methylacrylat, Hydroxyethylmethacrylat (HEMA), Glycidylmethacrylat, Glycidoxypropylmethacrylat, Glycerolmonomethacrylat (Isomerengemisch), N-Acryloxysuccinimid und Glycolmonomethacrylat.

12. Verwendung eines nukleinsäurebindenden Trägermaterials wie in einem oder mehreren der Ansprüche 1 bis 4 sowie 6 bis 9 definiert oder erhältlich nach einem der Ansprüche 10 oder 11, zur Aufreinigung von Nukleinsäuren.

## Claims

1. Method of purifying nucleic acids by means of binding and elution using a nucleic acid-binding phase, **characterized in that** the nucleic acid-binding phase comprises:
(i) nucleic acid-binding groups A having a pK from 8 to 13;
(ii) charge-neutral groups N having a neutral charge at the binding pH and the elution pH used;
wherein the nucleic acid-binding phase comprises a support material which is provided with different ligands (ligands I and ligands II), wherein the ligands I comprise at least one group A and the ligands II comprise at least one group N;
and wherein the method comprises at least the following steps:
(a) binding the nucleic acids to the nucleic acid-binding phase at a pH which is below the pK of the nucleic acid-binding groups A (binding pH);
(b) elution of the nucleic acids at a pH which is above the binding pH (elution pH).

2. Method according to claim 1, **characterized in that** the nucleic acid-binding phase is a modified support material, wherein said modification is selected from the group consisting of:
(i) modification of the support material with a mixture comprising at least the ligands I and II, wherein the ligands I comprise at least one group A and the ligands II comprise at least one group N;
(ii) modification of the support material with groups A and N, with the groups A being sterically shielded by compounds having at least one group N;
(iii) modification of the support material by a combination of the embodiments (i) and (ii).

3. Method according to claim 1 or 2, **characterized in that** the nucleic acid-binding phase comprises a support material which has one or more of the following features:
(i) the support material is an oxidic material; and/or
(ii) the support material is selected from the group consisting of Al₂O₃, TiO₂, ZrO₂, Ta₂O₅, SiO₂ and polysilicic acid; and/or
(iii) the support material is SiO₂ or polysilicic acid; and/or
(iv) the support material is an organic polymer, in particular selected from the group consisting of polystyrene and its derivatives, polyacrylate and polymethacrylate and its derivatives, polyurethane, nylon, polyethylene, polypropylene, polybutylidene and copolymers of these materials; and/or
(v) the support material is a polysaccharide, in particular a hydrogel such as agarose, cellulose, dextran, Sephadex, Sephacryl or chitosan; and/or
(vi) the support material is glass or metal; and/or
(vii) the support material is magnetic.

4. Method according to one or more of claims 1 to 3, **characterized in that** the nucleic acid-binding phase comprises groups A which have one or more of the following features:
(i) the groups A have a pK of from 9 to 13, preferably 10 to 12, particularly preferred 10 to 11; and/or
(ii) the groups A are tertiary, secondary or primary amino groups; and/or
(iii) the groups A form part of polymerizable monomers; and/or
(iv) the groups A are bound to a initiator molecule applied beforehand as a reactive silane; and/or
(v) the groups A were introduced as part of reactive silane groups; and/or
(vi) the groups A were introduced as part of compounds selected from the group consisting of: 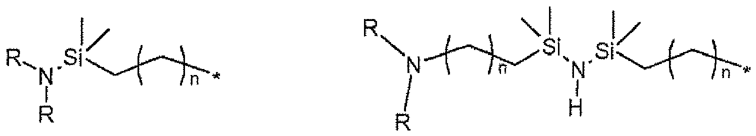 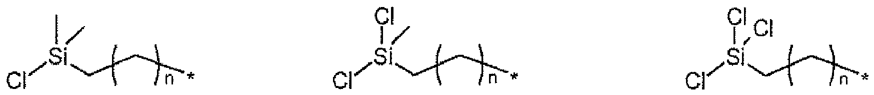 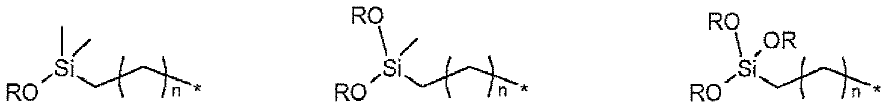 wherein
n is 1 to 5
R is a C1 to C6, preferably a C1 to C3, alkyl group; and
* is amino, aminomethyl, aminoethyl, aminopropyl, dimethylamino, diethylamino, diisopropylamino, dipropylamino, diethanolamino, dipropanolamino, diethylenetriamine, triethylenetetramine, tetraethylenepentamine, etheramine, polyetheramine, 4-diisobutylamino-1-butane, 6-dipropylamino-1-hexane; and/or
(vii) the groups A were introduced as part of compounds selected from the group consisting of diethylaminopropyl trimethoxysilane, dimethylaminopropyl trimethoxysilane and N,N-diisopropylaminopropyl trimethoxysilane.

5. Method according to one or more of claims 1 to 4, **characterized in that** the binding step (a) and/or the elution step (b) is carried out under conditions which fulfill one or more of the following features:
(i) in the elution step (b), the elution pH is below the pK of groups A; and/or
(ii) the salt concentration in the binding and/or elution buffer is from 1 mM to 1000 mM, 1 mM to 500 mM, 1 mM to 250 mM or 1 mM to 100 mM; and/or
(iii) the salt concentration is unchanged in the binding step (a) and/or in the elution step (b) or is raised during elution; and/or
(iv) the pH in the binding buffer is from pH 2 to pH 8, pH 2 to pH 7.5, pH 4 to pH 8 or pH 4 to pH 7.5; and/or
(v) the pH in the elution buffer is from pH 2 to pH 10, pH 4 to pH 10, pH 7 to pH 10 or pH 8 to pH 9; and/or
(vi) the temperature is unchanged in the binding step (a) and/or in the elution step (b) or is raised during elution; and/or
(vii) the temperature in the elution step is from 2°C to 95°C or 21°C to 60°C.

6. Method according to one or more of claims 1 to 5, **characterized in that** the groups N fulfill with one or more of the following features:
(i) the groups N are selected from the group consisting of hydroxyl groups, diol groups, triol groups, polyol groups, saccharides, epoxide groups, halides, alkyl groups, preferably C1-C6 alkyl groups, alkene groups, alkylene groups, imide groups, ether groups, or polyether groups, and/or
(ii) the groups N are charge-neutral in the pH ranges 2<pH<12; and/or
(iii) the groups N were introduced as part of polymerizable monomers; and/or
(iv) the groups N are bound to a initiator molecule applied previously as a reactive silane; and/or
(v) the groups N were introduced as part of reactive silane groups; and/or
(vi) the groups N were introduced as part of compounds selected from the group consisting of 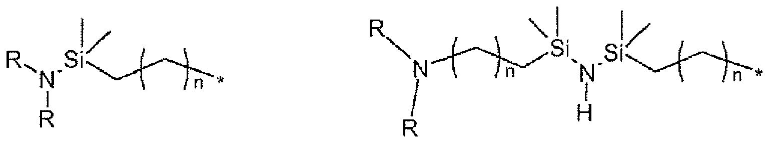 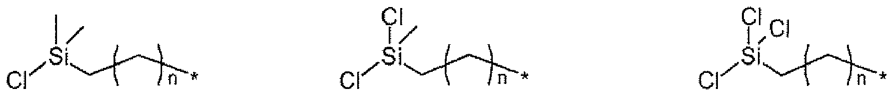 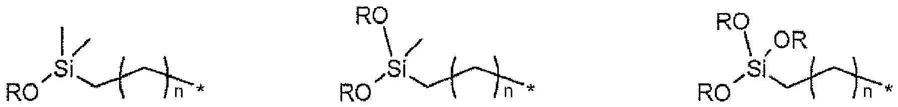
wherein
n is 1-5
R is a C1 to C6, preferably C1 to C3 alkyl group, in particular is methyl, ethyl, propyl, isopropyl;
* is amino, aminomethyl, aminoethyl, aminopropyl, dimethylamino, diethylamino, diisopropylamino, dipropylamino, diethanolamino, dipropanolamino, diethylenetriamine, triethylenetetramine, tetraethylenepentamine, etheramine, polyetheramine, 4-diisobutylamino-1-butane, 6-dipropylamino-1-hexane, hydroxymethyl, hydroxyethyl, hydroxypropyl, ethanediol, propanediol, propanetriol, butanetriol, 3-glycidoxypropyl, ethyl glycidyl ether, alkyl moiety, in particular a C1 to C4 alkyl moiety, in particular methyl, ethyl, propyl, isopropyl, butyl, isobutyl, halide or hydrogen.

7. Method according to one or more of claims 1 to 6, **characterized in that** it fulfills one or more of the following features:
(i) the support material was provided with the groups A and N by silanization; and/or
(ii) the groups A and/or N and/or T were introduced via reactive silanes; and/or
(iii) the groups A and/or N were introduced by monofunctional, bi- or trifunctional reactive silanes or a mixture of at least two reactive silanes with different functionality; and/or
(iv) the groups A and/or N were introduced by reactive silanes selected from the group consisting of aminosilanes, disilazanes, chlorosilanes or alkoxysilanes; and/or
(v) the proportion of groups A, related to the groups N, is from 1% to 99%, 1% to 50%, preferably 1% to 25%.

8. Method according to one or more of claims 2 to 7, **characterized by** one or more of the following features:
(i) the support material was modified with initiator molecules T, wherein subsequently the groups A and/or groups N were introduced in the form of monomers; and/or
(ii) the support material was modified with initiator molecules T, wherein said initiator molecules have one or more of the following features:
(aa) the initiator molecules are reactive silanes; and/or
(bb) the initiator molecules are selected from the group consisting of 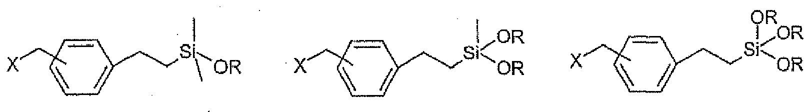 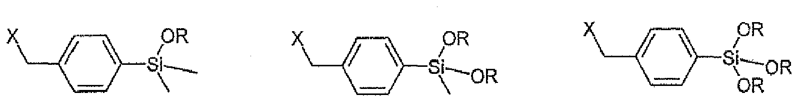 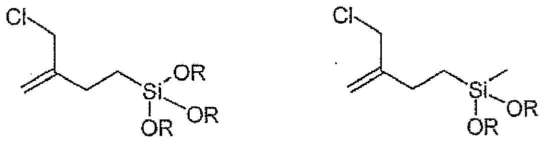 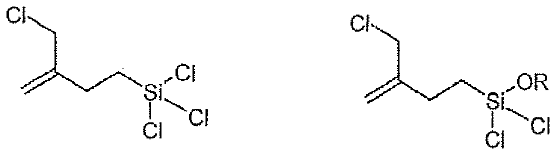 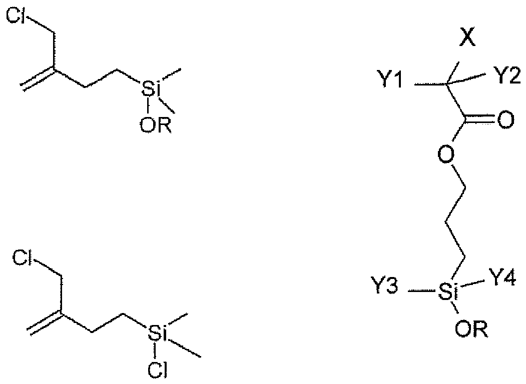
wherein X is halogen, in particular Cl, Br, I
Y1, Y2, Y3 or Y4 are, independently of one another R, OR, OH or H
and R is C1-C3 alkyl:
(iii) the initiator molecule is 2-(chloromethyl)allyl trimethoxysilane;
(iv) the initiator molecule is [3-(2-bromoisobutyryl)propyl]ethoxydimethylsilane;
(v) the groups A were introduced by monomers selected from the group consisting of
N-(3-aminomethyl)methacrylamide, N-(3-aminoethyl)methacrylamide,
N-(3-aminopropyl)methacrylamide, N-(3-aminoisopropyl)methacrylamide,
N,N-dimethylacrylamide, N,N-diethylacrylamide, N,N-diisopropylacrylamide,
N,N-(dimethylamino)ethylacrylamide, N,N-(dimethylamino)ethyl acrylate,
N,N-(dimethylamino)ethylmethacrylamide, N,N-(dimethylamino)ethyl methacrylate,
N,N-(dimethylamino)propylacrylamide, N,N-(dimethylamino)propyl acrylate,
N,N-(dimethylamino)propylmethacrylamide,
N,N-(dimethylamino)propyl methacrylate, N,N-(diethylamino)ethylacrylamide,
N,N-(diethylamino)ethyl acrylate, N,N-(diethylamino)ethylmethacrylamide,
N,N-(diethylamino)ethyl methacrylate, N,N-(diethylamino)propylacrylamide,
N,N-(diethylamino)propyl acrylate, N,N-(diethylamino)propylmethacrylamide,
N,N-(diethylamino)propylmethacrylate, N,N-(diisopropylamino)ethylacrylamide,
N,N-(diisopropylamino)ethyl acrylate, N,N-(diisopropylamino)ethylmethacrylamide,
N,N-(diisopropylamino)ethyl methacrylate,
N,N-(diisopropylamino)propylacrylamide, N,N-(diisopropylamino)propyl acrylate,
N,N(dimethylamino)propylmethacrylamide,
N,N-(dimethylamino)propyl methacrylate, 2-(diisopropylamino)ethylmethacrylate and/or
(vi) the groups N were introduced by monomers selected from the group consisting of butyl acrylate, propyl acrylate, ethyl acrylate, methyl acrylate, glycidyl methacrylate, hydroxyethyl methacrylate (HEMA), glycidoxypropyl methacrylate, glycerolmono-methacrylate (isomeric mixture), glycol mono-methacrylate, glycidyl acrylate, hydroxyethyl acrylate, glycidoxypropyl acrylate, glycerol mono-acrylate (isomeric mixture), glycol mono-acrylate and N-acryloxysuccinimide.

9. Method according to one or more of claims 1 o 8, **characterized in that** the support material comprises initiator groups T which are at least partially functionalized with compounds comprising groups A, said groups A being sterically shielded by groups N, said steric shielding being generated by one or more of the following modifications:
(i) the groups N are in the form of chain-shaped branched or unbranched oligomers or polymers which sterically shield the groups A; and/or
(ii) the groups N are in the form of oligomers or polymers composed of monomers, which have a chain length of n=10-1000, preferably n=10-100; and/or
(iii) the groups N are present in form of polyacrylate, preferably according to the following formula: 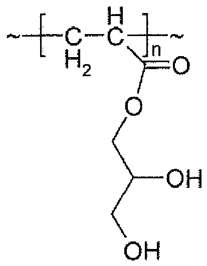
(iv) the groups N possess respectively are spatially extensive groups such as, for example, isopropyl, isobutyl, tert-butyl or n-hexyl moieties.

10. Method of preparing a nucleic acid-binding support material as defined in one or more of claims 1 to 4 and 6 to 9, said method being carried out according to at least one of the following alternatives (A) to (D):
Alternative (A): Functionalization of the support material with a mixture comprising at least the ligands I and II, wherein the ligands I comprise at least one group A and the ligands II comprise at least one group N; and/or
Alternative (B): (a) Functionalization of the support material with a mixture of initiator molecules T and ligands comprising at least one group A (b) Addition of compounds comprising at least one group N and attachment of said compounds to the initiator molecules T; and/or
Alternative (C): Functionalization of the support material with groups A and N, with the groups A being sterically shielded by compounds comprising at least one group N; and/or
Alternative (D): Functionalization of the support material by a combination of at least two of the alternatives (A) to (C).

11. Method according to claim 10, **characterized in that** it fulfills at least one of the following features:
(i) the groups A are tertiary, secondary or primary amino groups; and/or
(ii) the groups N are selected from the group consisting of hydroxyl groups, diol groups, triol groups, polyol groups, saccharides, epoxide groups, halides, C1-C6 alkyl groups, alkene groups, alkylene groups, imide groups, ether groups, or polyether groups and/or
(iii) the groups N are charge-neutral at the elution pH and preferably in the specified pH ranges of 2<pH<12; and/or
(iv) the groups A and/or N are introduced by monomers; and/or
(v) the groups A and/or N are introduced by silanization; and/or
(vi) the groups A and/or N are introduced by reactive silanes; and/or
(vii monofunctional or bi- and/or trifunctional reactive silanes are used to introduce the groups A and/or N; and/or
(viii) reactive silanes selected from the group consisting of amino silanes, disilazanes, chlorosilanes or alkoxysilanes are used to introduce the groups A and/or N; and/or
(ix) the groups A and/or N are introduced by reactive silanes selected from the group consisting of: 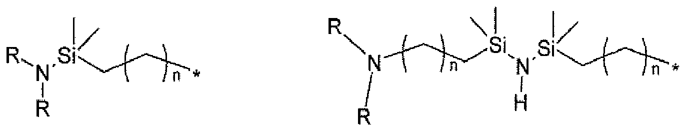 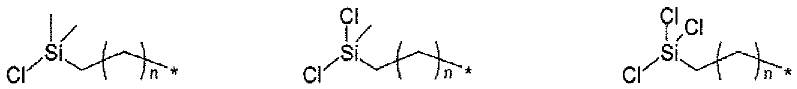 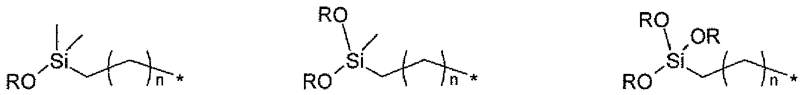 wherein
n is 1-5
R is a C1 to C6, preferably C1 to C3, alkyl group, in particular methyl, ethyl, propyl, isopropyl;
* is amino, aminomethyl, aminoethyl, aminopropyl, dimethylamino, diethylamino, diisopropylamino, dipropylamino, diethanolamino, dipropanolamino, diethylenetriamine, triethylenetetramine, tetraethylenepentamine, etheramine, polyetheramine, 4-diisobutylamino-1-butane, 6-dipropylamino-1-hexane, hydroxymethyl, hydroxyethyl, hydroxypropyl, ethanediol, propanediol, propanetriol, butanetriol, 3-glycidoxypropyl, ethyl glycidyl ether, alkyl moiety, in particular a C1 to C4 alkyl moiety, in particular methyl, ethyl, propyl, isopropyl, butyl, isobutyl, halide or hydrogen;
(x) the groups A and N are introduced in such a ratio that the proportion of the A groups is from 1% to 99%,1% to 50%, preferably 1 %to 25%: and/or
(xi) the support material is modified with initiator molecules T having one or more of the following features:
(aa) the initiator molecules are applied by silanization; and/or
(bb) the initiator molecules are silanes; and/or
(cc) the initiator molecules are selected from the group consisting of 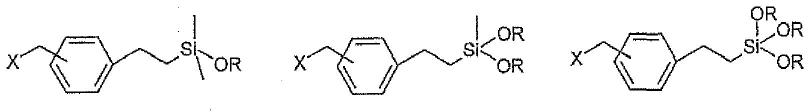 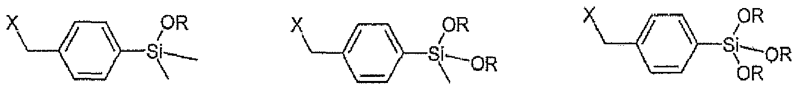 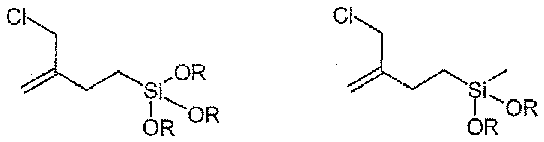 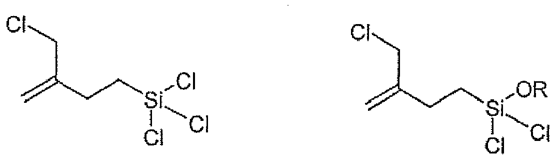 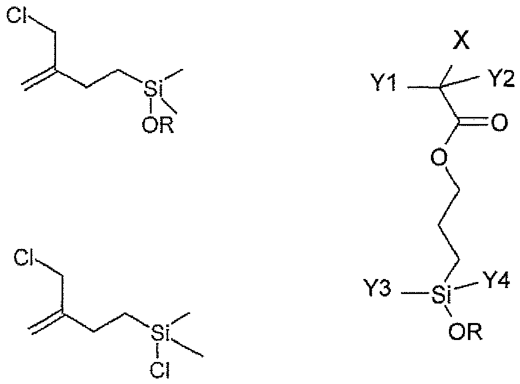
wherein X is halogen, in particular Cl, Br, I
Y1, Y2, Y3 or Y4 are, independently of one another, R, OR, OH or H
and R is C1-C3 alkyl.
(xii) the initiator molecule used is 2-(chloromethyl)allyl trimethoxysilane;
(xiii)the initiator molecule used is [3-(2-bromoisobutyryl)propyl]ethoxydimethylsilane; and/or
(xiv)the groups A are introduced by monomers selected from the group consisting of
2-(diisopropylamino)ethylmethacrylate, N-(3-aminomethyl)methacrylamide,
N-(3-aminoethyl)methacrylamide, N-(3-aminopropyl)methacrylamide,
N-(3-aminoisopropyl)methacrylamide, N,N-dimethylacrylamide,
N,N-diethylacrylamide, N,N-diisopropylacrylamide,
N,N-(dimethylamino)ethylacrylamide, N,N-(dimethylamino)ethyl acrylate,
N,N-(dimethylamino)ethylmethacrylamide, N,N-(dimethylamino)ethyl methacrylate,
N,N-(dimethylamino)propylacrylamide, N,N-(dimethylamino)propyl acrylate,
N,N-(dimethylamino)propylmethacrylamide,
N,N-(dimethylamino)propyl methacrylate, N,N-(diethylamino)ethylacrylamide,
N,N-(diethylamino)ethyl acrylate, N,N-(diethylamino)ethylmethacrylamide,
N,N-(diethylamino)ethyl methacrylate, N,N-(diethylamino)propylacrylamide,
N,N-(diethylamino)propyl acrylate, N,N-(diethylamino)propylmethacrylamide,
N,N-(diethylamino)propylmethacrylate, N,N-(diisopropylamino)ethylacrylamide,
N,N-(diisopropylamino)ethyl acrylate, N,N-(diisopropylamino)ethylmethacrylamide,
N,N-(diisopropylamino)ethyl methacrylate,
N,N-(diisnpropylamino)propylacrylamide, N,N-(diisopropylamino)propyl acrylate,
N,N(dimethylamino)propylmethacrylamide,
N,N-(dimethylamino)propyl methacrylate and/or
(xv) the groups N are introduced by monomers selected from the group consisting of butyl acrylate, propyl acrylate, ethyl acrylate, methyl acrylate, hydroxyethyl methacrylate (HEMA), glycidyl methacrylate, glycidoxypropyl methacrylate, glycerolmono-methacrylate (isomeric mixture), N-acryloxysuccinimide and glycol mono-methacrylate.

12. Use of a nucleic acid-binding support material as defined in one or more of claims 1 to 4 and 6 to 9 or obtainable by one of claims 10 or 11 for purifying nucleic acids.

## Revendications

1. Procédé de purification d'acides nucléiques au moyen d'une phase liant les acides nucléiques par liaison et élution, **caractérisé en ce que** la phase liant les acides nucléiques présente:
(i) des groupes liant les acides nucléiques A, lesquels présentent un pK de 8 à 13;
(ii) des groupes de charge neutre N, lesquels présentent une charge neutre au pH de liaison utilisé et au pH d'élution utilisé;
la phase liant les acides nucléiques présentant un matériau de support qui est pourvu de différents ligands (ligands I et ligands II), les ligands présentant au moins un groupe A et les ligands II au moins un groupe N;
et le procédé présentant au moins les étapes suivantes:
(a)liaison des acides nucléiques à la phase liant les acides nucléiques à un pH qui est inférieur au pK des groupes liant les acides nucléiques A (pH de liaison);
(b)élution des acides nucléiques à un pH qui est supérieur au pH de liaison (pH d'élution).

2. Procédé selon la revendication 1, **caractérisé en ce que** la phase liant les acides nucléiques est un matériau de support modifié, la modification étant sélectionnée dans le groupe constitué de:
(i) modification du matériau de support avec un mélange présentant au moins les ligands I et II, les ligands I présentant au moins un groupe A et les ligands II au moins un groupe N;
(ii) modification du matériau de support avec des groupes A et N, les groupes A étant protégés stériquement par des composés qui présentent au moins un groupe N;
(iii) modification du matériau de support par une combinaison des modes de réalisation (i) et (ii).

3. Procédé selon la revendication 1 ou 2, **caractérisé en ce que** la phase liant les acides nucléiques présente un matériau de support qui présente une ou plusieurs des caractéristiques suivantes:
(i) le matériau de support est un matériau oxydique; et/ou
(ii) le matériau de support est sélectionné dans le groupe constitué de Al₂O₃, TiO₂, ZrO₂, Ta₂O₅, SiO₂ et de l'acide polysilicique; et/ou
(iii) le matériau de support est SiO₂ ou de l'acide polysilicique; et/ou
(iv) le matériau de support est un polymère organique, en particulier sélectionné dans le groupe constitué de: polystyrène et ses dérivés, polyacrylate et -méthacrylate et ses dérivés, polyuréthane, nylon, polyéthylène, polypropylène, polybutylidène et des copolymères de ces matériaux; et/ou
(v) le matériau de support est un polysaccharide, en particulier un hydrogel tel que agarose, cellulose, dextrand, séphadex, séphacryl ou chitosane; et/ou
(vi) le matériau de support est du verre ou du métal; et/ou
(vii) le matériau de support est magnétique.

4. Procédé selon l'une ou plusieurs des revendications 1 à 3, **caractérisé en ce que** la phase liant les acides nucléiques présente des groupes A qui présentent une ou plusieurs des caractéristiques suivantes:
(i) les groupes A présentent un pK de 9 à 13, de préférence de 10 à 12, de manière particulièrement préférée de 10 à 11; et/ou
(ii) les groupes A sont des groupes amino tertiaires, secondaires ou primaires; et/ou
(iii) les groupes A sont des constituants de monomères polymérisables; et/ou
(iv) les groupes A sont liés à une molécule initiatrice préalablement apportée sous la forme de silane réactif; et/ou
(v) les groupes A ont été introduits en tant que constituant de groupes silane réactifs; et/ou
(vi) les groupes A ont été introduits en tant que constituant de composés qui sont sélectionnés dans le groupe constitué de: 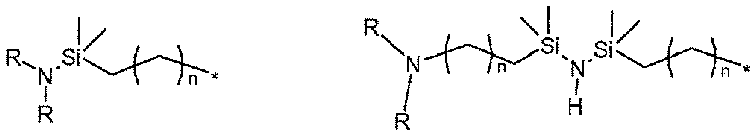 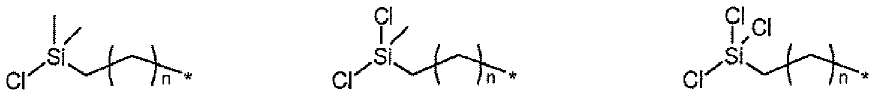 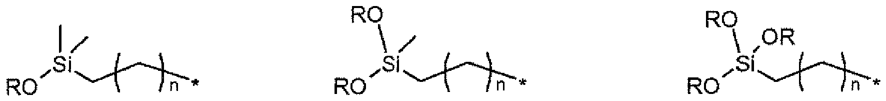 Où
n: 1 à 5
R est un groupe alkyle C1 à C6, de préférence C1 à C3; et
* est amino-, aminométhyl-, aminoéthyl-, aminopropyl-, diméthylamino-, diéthylamino-, diisopropylamino-, dipropylamino-, diéthanolamino-, dipropanolamino-, diéthylènetriamine-, triéthylènetétramine-, tétraéthylènepentamine, éthéramine, polyétheramine, 4-diisobutylamino-1-butane, 6-dipropylamino-1-hexane; et/ou
(vii) les groupes A ont été introduits en tant que constituant de composés qui sont sélectionnés dans le groupe constitué de: diéthylamino-propyltriméthoxysilane, diméthylamino-propyltriméthoxysilane et N,N-diisopropylaminopropyltriméthoxysilane.

5. Procédé selon l'une ou plusieurs des revendications 1 à 4, **caractérisé en ce que** l'étape de liaison (a) et/ou l'étape d'élution (b) sont réalisées dans des conditions qui présentent une ou plusieurs des caractéristiques suivantes:
(i) à l'étape d'élution (b), le pH d'élution est inférieur au pK des groupes A; et/ou
(ii) la concentration en sel dans le tampon de liaison et/ou d'élution est de 1 mM à 1000 mM, 1 mM à 500 mM, 1 mM à 250 mM ou 1 mM à 100 mM; et/ou
(iii) la concentration en sel est inchangée à l'étape de liaison (a) et/ou à l'étape d'élution (b) ou est augmentée lors de l'élution; et/ou
(iv) le pH dans le tampon de liaison est de pH 2 à pH 8, de pH 2 à pH 7,5, de pH 4 à pH 8 ou de pH 4 à pH 7,5 et/ou
(v) le pH dans le tampon d'élution est de pH 2 à pH 10, de pH 4 à pH 10, de pH 7 à pH 10 ou de pH 8 à pH 9; et/ou
(vi) la température est inchangée à l'étape de liaison (a) et/ou à l'étape d'élution (b) ou est augmentée lors de l'élution; et/ou
(vii) la température à l'étape d'élution est de 2 °C à 95 °C ou de 21 °C à 60 °C.

6. Procédé selon l'une ou plusieurs des revendications 1 à 5, **caractérisé en ce que** les groupes N présentent une ou plusieurs des caractéristiques suivantes:
(i) les groupes N sont sélectionnés dans le groupe constitué des groupes hydroxyle, groupes diol, groupes triol, groupes polyol, saccharides, groupes époxy, halogénures, groupes alkyle, de préférence groupes alkyle C1-C6, groupes alcène, groupes alkylène, groupes imide, groupes éther ou groupes polyéther et/ou
(ii) les groupes N sont de charge neutre dans la plage de pH 2 < pH < 12; et/ou
(iii) les groupes N ont été introduits en tant que constituant de monomères polymérisables; et/ou
(iv) les groupes N sont présents liés à une molécule initiatrice préalablement apportée sous la forme de silane réactif; et/ou
(v)les groupes N ont été introduit en tant que constituant de groupes silane réactifs; et/ou
(vi) les groupes N ont été introduit en tant que constituant de composés qui sont sélectionnés dans le groupe constitué de: 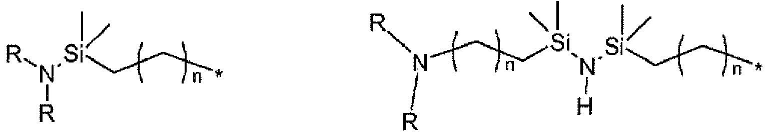 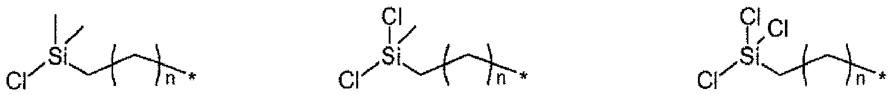 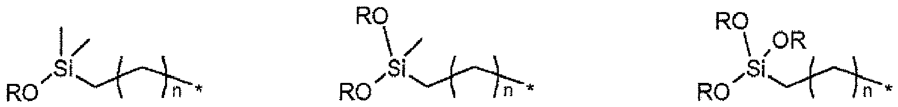
où
n= 1-5
R est un groupe alkyle C1 à C6, de préférence C1 à C3, en particulier méthyle, éthyle, propyle, i-propyle;
* est amino-, aminométhyl-, aminoéthyl-, aminopropyl-, diméthylamino-, diéthylamino-, diisopropylamino-, dipropylamino-, diéthanolamino-, dipropanolamino-, diéthylènetriamine, triéthylènetétramine, tétraéthylènepentamine, éthéramine, polyétheramine, 4-diisobutylamino-1-butane, 6-dipropylamino-1-hexane, hydroxyméthyl-, hydroxyéthyl-, hydroxypropyl-, éthanediol-, propanediol-, propanetriol-, butanetriol, 3-glycidoxypropyl, éthylglycidyléther, reste alkyle, en particulier un reste alkyle C1 à C4, en particulier méthyle, éthyle, propyle, i-propyle, butyle, i-butyle, un halogénure ou de l'hydrogène.

7. Procédé selon l'une ou plusieurs des revendications 1 à 6, **caractérisé en ce qu'**une ou plusieurs des conditions suivantes sont remplies:
(i) le matériau de support a été pourvu des groupes A et N par silanisation; et/ou
(ii) les groupes A et/ou N et/ou T ont été introduits par des silanes réactifs; et/ou
(iii) les groupes A et/ou N ont été introduits par des silanes réactifs monofonctionnels, bi-ou trifonctionnels ou un mélange d'au moins deux silanes réactifs de fonctionnalité différente; et/ou
(iv) les groupes A et/ou N ont été introduits par des silanes réactifs qui sont sélectionnés dans le groupe constitué des aminosilanes, disilazanes, chlorosilanes ou alkoxysilanes; et/ou
(v) la proportion des groupes A par rapport aux groupes N est de 1 % à 99 %, de 1 % à 50 %, de préférence de 1 % à 25 %.

8. Procédé selon l'une ou plusieurs des revendications 2 à 7, **caractérisé par** une ou plusieurs des caractéristiques suivantes:
(i) le matériau de support a été modifié par des molécules initiatrices T, les groupes A et/ou les groupes N ayant été introduits à la suite sous la forme de monomères; et/ou
(ii) le matériau de support a été modifié par des molécules initiatrices T, les molécules initiatrices présentant une ou plusieurs des caractéristiques suivantes:
(aa) les molécules initiatrices sont des silanes réactifs; et/ou
(bb) les molécules initiatrices sont sélectionnées dans le groupe constitué de: 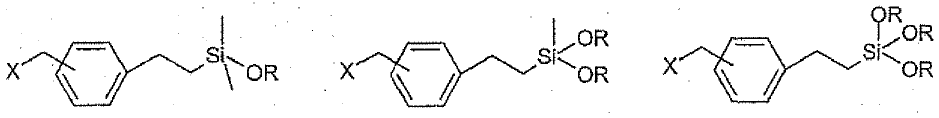 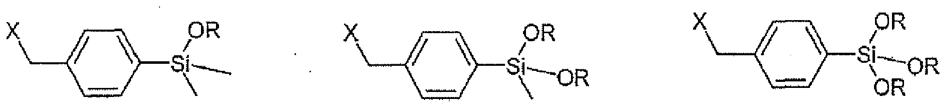 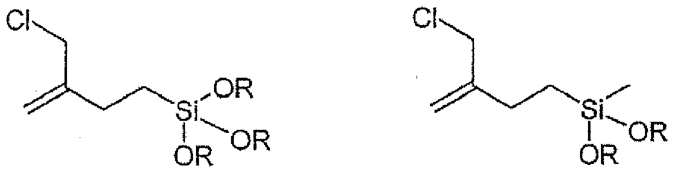 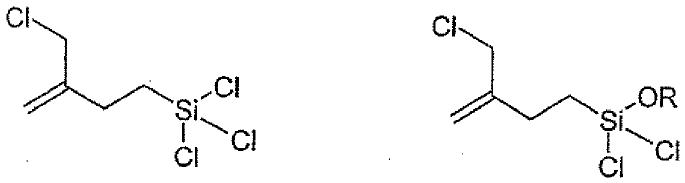 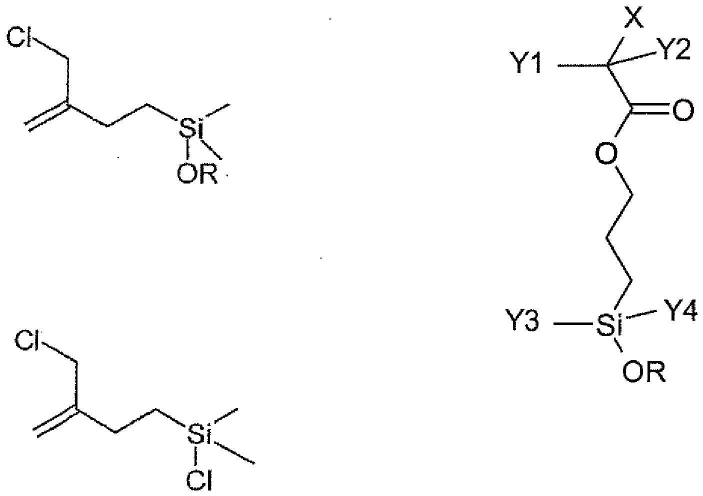
Où X = halogène, en particulier Cl, Br, I
Y1, Y2, Y3 ou Y4 = indépendamment l'un de l'autre R, OR, OH ou H et R = alkyle C1 - C3.
(iii) la molécule initiatrice est 2-(chlorométhyl)allyltriméthoxysilane;
(iv) la molécule initiatrice est [3-(2-bromoisobutyryl)propyl]-éthoxydiméthylsilane;
(v) les groupes A ont été introduits par des monomères qui sont sélectionnés dans le groupe constitué de:
N-(3-aminométhyl)méthacrylamide, N-(3-aminoéthyl)méthacrylamide, N-(3-aminopropyl)méthacrylamide, N-(3-aminoisopropyl)méthacrylamide, N,N-diméthylacrylamide, N,N-diéthylacrylamide, N,N-diisopropylacrylamide, N,N-(diméthylamino)éthylacrylamide, N,N-(diméthylamino)éthylacrylate, N,N-(diméthylamino)éthylméthacrylamide, N,N-(diméthylamino)éthylméthacrylate, N,N-(diméthylamino)propylacrylamide, N,N-(diméthylamino)propylacrylate, N,N-(diméthylamino)propylméthacrylamide, N,N-(diméthylamino)propylméthacrylate, N,N-(diéthylamino)éthylacrylamide, N,N-(diéthylamino)éthylacrylate, N,N-(diéthylamino)éthylméthacrylamide, N,N-(diéthylamino)éthylméthacrylate, N,N-(diéthylamino)propylacrylamide,N,N-(diéthylamino)propylacrylate, N,N-(diéthylamino)propylméthacrylamide, N,N-(diéthylamino)propylméthacrylate, N,N-(diisopropylamino)éthylacrylamide, N,N-(diisopropylamino)éthylacrylate, N,N-(diisopropylamino)éthylméthacrylamide, N,N-(diisopropylamino)éthylméthacrylate, N,N-(diisopropylamino)propylacrylamide, N,N-(diisopropylamino)propylacrylate, N,N-(diméthylamino)propylméthacrylamide, N,N-(diméthylamino)propylméthacrylate, 2-(diisopropylamino)éthylméthacrylate
et/ou
(vi) les groupes N ont été introduits par des monomères qui sont sélectionnés dans le groupe constitué de : butylacrylate, propylacrylate, éthylacrylate, méthylacrylate, glycidyle-méthacrylate, hydroxyéthyle méthacrylate (HEMA), glycidoxypropyle méthacrylate, glycérol monométhacrylate (mélange d'isomères), glycol monométhacrylate, glycidylacrylate, hydroxyéthylacrylate, glycidoxypropyle acrylate, glycérol monoacrylate (mélange d'isomères), glycol monoacrylate et N-acryloxysuccinimide.

9. Procédé selon l'une ou plusieurs des revendications 1 à 8, **caractérisé en ce que** le matériau de support présente des groupes initiateurs T qui sont fonctionnalisés au moins en partie par des composés qui présentent des groupes A, les groupes A étant protégés stériquement par des groupes N, la protection stérique étant produite par une ou plusieurs des modifications suivantes:
(i) les groupes N sont présents sous la forme d'oligomères ou de polymères linéaires ramifiés ou non ramifiés, qui protègent stériquement les groupes A; et/ou
(ii) les groupes N sont présents sous la forme d'oligomères ou de polymères formés de monomères, ceux-ci présentant une longueur de chaîne de n = 10 - 1000, de préférence n = 10 - 100; et/ou
(iii) les groupes N sont présents sous la forme de polyacrylate, de préférence selon la formule suivante: 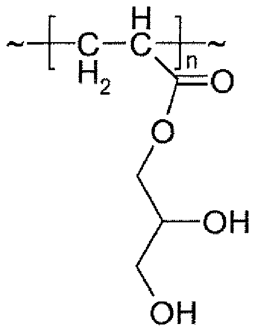
(iv) les groupes N possèdent ou sont des groupes spatialement étendus comme par exemple isopropyle, isobutyle, tert.-butyle ou restes n-hexyle.

10. Procédé de fabrication d'un matériau de support liant les acides nucléiques tel que défini dans une ou plusieurs des revendications 1 à 4 ainsi que 6 à 9, lequel procédé est mis en oeuvre selon au moins l'une des alternatives suivantes (A) à (D):
Alternative (A): fonctionnalisation du matériau de support avec un mélange présentant au moins les ligands I et II, les ligands I présentant au moins un groupe A et les ligands Il présentant au moins un groupe N; et/ou
Alternative (B): (a) fonctionnalisation du matériau de support avec un mélange de molécules initiatrices T et de ligands qui présentent au moins un groupe A (b) ajout de composés présentant au moins un groupe N et liaison de ceux-ci aux molécules initiatrices T; et/ou
Alternative (C): fonctionnalisation du matériau de support avec des groupes A et N, les groupes A étant protégés stériquement par des composés qui présentent au moins un groupe N ; et/ou
Alternative (D): fonctionnalisation du matériau de support par une combinaison d'au moins deux des alternatives (A) à (C).

11. Procédé selon la revendication 12, **caractérisé en ce qu'**il présente au moins une des caractéristiques suivantes:
(i) les groupes A sont des groupes amino tertiaires, secondaires ou primaires; et/ou
(ii) les groupes N sont sélectionnés dans le groupe constitué des groupes hydroxyle, groupes diol, groupes triol, groupes polyol, saccharides, groupes époxy, halogénures, groupes alkyle C1-C6, groupes alcène, groupes alkylène, groupes imide, groupes éther ou groupes polyéther et/ou
(iii) les groupes N sont de charge neutre au pH d'élution et de préférence dans la plage de pH spécifiée 2 < pH < 12; et/ou
(iv) les groupes A et/ou N sont introduits par des monomères; et/ou
(v) les groupes A et/ou N sont introduits par silanisation; et/ou
(vi) les groupes A et/ou N sont introduits par des silanes réactifs; et/ou
(vii) des silanes réactifs monofonctionnels, bi- et/ou trifonctionnels sont utilisés pour introduire les groupes A et/ou N; et/ou
(viii) des silanes réactifs qui sont sélectionnés dans le groupe constitué des aminosilanes, disilazanes, chlorosilanes ou alkoxysilanes sont utilisés pour introduire les groupes A et/ou N; et/ou
(ix) les groupes A et/ou N sont introduits par des silanes réactifs qui sont sélectionnés dans le groupe constitué de: 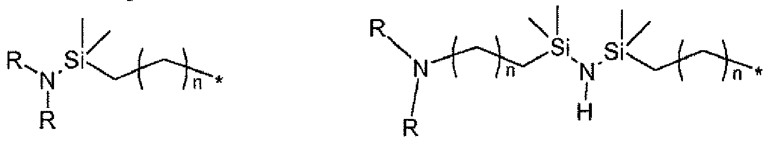 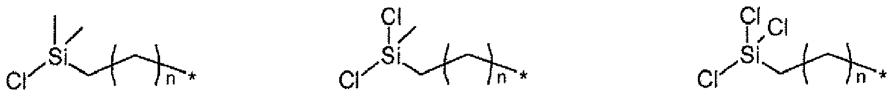 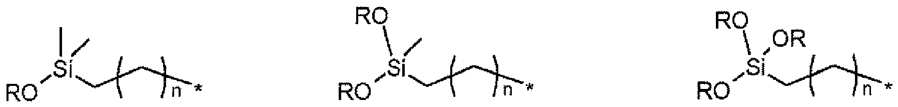 où
n= 1-5
R est un groupe alkyle C1 à C6, de préférence C1 à C3, en particulier méthyle, éthyle, propyle, i-propyle;
* est amino-, aminométhyl-, aminoéthyl-, aminopropyl-, diméthylamino-, diéthylamino-, diisopropylamino-, dipropylamino-, diéthanolamino-, dipropanolamino-, diéthylènetriamine, triéthylènetétramine, tétraéthylènepentamine, éthéramine, polyétheramine, 4-diisobutylamino-1-butane, 6-dipropylamino-1-hexane, hydroxyméthyl-, hydroxyéthyl-, hydroxypropyl-, éthanediol-, propanediol-, propanetriol-, butanetriol, 3-glycidoxypropyl, éthylglycidyléther, reste alkyle, en particulier un reste alkyle C1 à C4, en particulier méthyle, éthyle, propyle, i-propyle, butyle, i-butyle, un halogénure ou de l'hydrogène.
(x) les groupes A et N sont introduits dans un rapport tel que la proportion des groupes A soit de 1 % à 99 %, de 1 % à 50 %, de préférence de 1 % à 25 %; et/ou
(xi) le matériau de support est modifié par des molécules initiatrices T qui présentent une ou plusieurs des caractéristiques suivantes:
(aa) les molécules initiatrices sont déposées par silanisation; et/ou
(bb) les molécules initiatrices sont des silanes; et/ou
(cc) les molécules initiatrices sont sélectionnées dans le groupe constitué de: 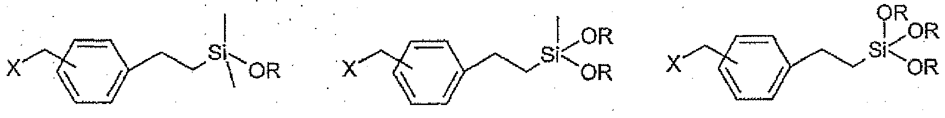 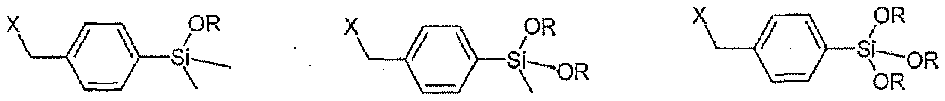 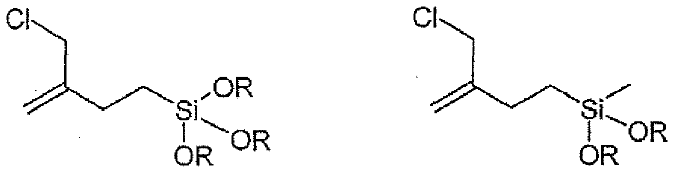 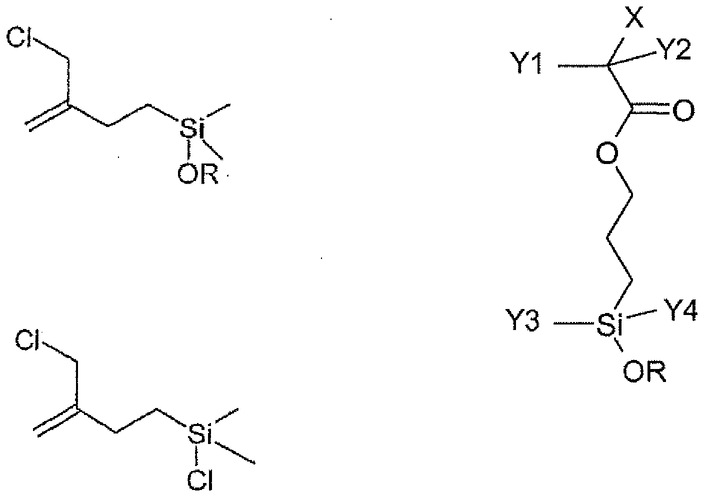
Où X = halogène, en particulier Cl, Br, I
Y1, Y2, Y3 ou Y4 = indépendamment l'un de l'autre R, OR, OH ou H et R = alkyle C1 - C3.
(xii) on utilise comme molécule initiatrice 2-(chlorométhyl)allyltriméthoxysilane;
(xiii)on utilise comme molécule initiatrice [3-(2-bromoisobutyryl)propyl]-éthoxydiméthylsilane; et/ou
(xiv) les groupes A sont introduits par des monomères qui sont sélectionnés dans le groupe constitué de:
2-(diisopropylamino)éthylméthacrylate, N-(3-aminométhyl)méthacrylamide, N-(3-aminoéthyl)méthacrylamide, N-(3-aminopropyl)méthacrylamide, N-(3-aminoisopropyl)méthacrylamide, N,N-diméthylacrylamide, N,N-diéthylacrylamide, N,N-diisopropylacrylamide, N,N-(diméthylamino)éthylacrylamide, N,N-(diméthylamino)éthylacrylate, N,N-(diméthylamino)éthylméthacrylamide, N,N-(diméthylamino)éthylméthacrylate, N,N-(diméthylamino)propylacrylamide, N,N-(diméthylamino)propylacrylate, N,N-(diméthylamino)propylméthacrylamide, N,N-(diméthylamino)propylméthacrylate, N,N-(diéthylamino)éthylacrylamide, N,N-(diéthylamino)éthylacrylate, N,N-(diéthylamino)éthylméthacrylamide, N,N-(diéthylamino)éthylméthacrylate, N,N-(diéthylamino)propylacrylamide,N,N-(diéthylamino)propylacrylate, N,N-(diéthylamino)propylméthacrylamide, N,N-(diéthylamino)propylméthacrylate, N,N-(diisopropylamino)éthylacrylamide, N,N-(diisopropylamino)éthylacrylate, N,N-(diisopropylamino)éthylméthacrylamide, N,N-(diisopropylamino)éthylméthacrylate, N,N-(diisopropylamino)propylacrylamide, N,N-(diisopropylamino)propylacrylate, N,N-(diméthylamino)propylméthacrylamide, N,N-(diméthylamino)propylméthacrylate, et/ou
(xiv) les groupes N sont introduits par des monomères qui sont sélectionnés dans le groupe constitué de: butylacrylate, propylacrylate, éthylacrylate, méthylacrylate, hydroxyéthyle méthacrylate (HEMA), glycidyle-méthacrylate, glycidoxypropyle méthacrylate, glycérol monométhacrylate (mélange d'isomères), N-acryloxysuccinimide et glycol monométhacrylate.

12. Utilisation d'un matériau de support liant les acides nucléiques, tel que défini dans une ou plusieurs des revendications 1 à 4 ainsi que 6 à 9 ou pouvant être obtenu selon l'une des revendications 10 ou 11, pour purifier des acides nucléiques.
